# EUROPEAN PATENT APPLICATION

(11) **EP 1 640 014 A1**
(43) Date of publication of application: **29.03.2006**
(21) Application number: 04733155.8
(22) Date of filing: 14.05.2004
(51) Int. Cl.: A61K 36/00, A23L 1/30, A23L 2/00, A23K 1/16, A61P 3/10

(54) **REMEDY**

(30) Priority: 19.05.2003 JP 2003140802
(71) Applicant: TAKARA BIO INC., Otsu-shi, Shiga 520-2193 (JP)
(72) Inventor: ENOKI, Tatsuji, Kyotanabe-shi, Kyoto 610-0313 (JP); OGAWA, Kinuko, Otsu-shi, Shiga 520-2134 (JP); OHNOGI, Hiromu, Kusatsu-shi, Shiga 525-0025 (JP); SUGIYAMA, Katsumi, Otsu-shi, Shiga 520-2134 (JP); MURAKI, Nobuko, Koka-shi, Shiga 529-1851 (JP); KUDO, Yoko, Otsu-shi, Shiga 520-2134 (JP); SAGAWA, Hiroaki, Kusatsu-shi, Shiga 525-0025 (JP); KATO, Ikunoshin, Koka-shi, Shiga 529-1851 (JP)
(74) Representative: Vossius & Partner
(86) International application number: PCT/JP2004/006874
(87) International publication number: WO 2004/100969

(57) **Abstract**

The present invention relates to a therapeutic agent and prophylactic agent for a disease accompanying an abnormality in an amount of insulin or insulin response, an agent for an insulin-mimetic action, a food, beverage and feed for treating a preventing a disease accompanying an abnormality in an amount of insulin or insulin response, an agent for enhancing glucose uptake into a cell, and an agent for inducing differentiation into an adipocyte, **characterized in that** each comprises as an effective ingredient a processed product derived from at least one plant selected from the group consisting of (a) a plant belonging to Tiliaceae, (b) a plant belonging to Zingiberaceae, (c) a plant belonging to Compositae, (d) a plant belonging to Liliaceae, and (e) a plant belonging to Polygonaceae.

## Description

### TECHNICAL FIELD

The present invention relates to a medicament, food, beverage or feed which is useful in treating or preventing a disease associated with insulin in a living body, for example, diabetes, obesity or the like.

### BACKGROUND ART

Insulin is a hormone necessary for normal metabolism of carbohydrates, proteins and fats in a mammal. Since human suffering from type I diabetes does not sufficiently produce insulin, which is a hormone sustaining life, administration of insulin from the external is required for survival. Human suffering from type II diabetes needs administering with insulin or an agent for enhancing insulin secretion in order to control the glucose level in blood from an inappropriate level, due to causations such as deficiency of the amount of insulin produced and insulin resistance, to an appropriate level. However, among humans suffering from type II diabetes, therapeutic effects may not be found in cases where insulin or the agent for enhancing insulin secretion is administered to diabetic patients of which cause is insulin resistance caused by hyperinsulinemia, abnormality in insulin receptor, aberrance in a downstream signal of the insulin receptor or the like.

In recent years, in order to solve the side effects of insulin and the above-mentioned problems, developments have been made on a substance having physiological functions similar to those of insulin (hereinafter referred to as insulin-mimetic substance in some cases). It has been found that a synthetic benzoquinone derivative is an insulin-mimetic substance (for example, WO 99/51225), and that shikonin derived from *Lithospermum erythrorhizon* is an insulin-mimetic substance (for example, Kamei R. and seven others, *Biochem. Biophys. Res. Commun.,* 2002, Vol. 292, P642-651). These insulin-mimetic substances as mentioned above have been expected to ameliorate symptoms by exhibiting physiological activities similar to those of insulin, not only in type I diabetic patients but also in type II diabetic patients, as well as in type II diabetic patients of which cause is insulin resistance.

Mulukhiya, which has a nomenclature of *Corchorus olitorius,* is an annual herb belonging to Dicotyledoneae Tiliaceae *Corchorus* (Japanese name: Tsunaso). The Mulukhiya has been known as a green-yellow vegetable which is native to Egypt and nutrious, and as its pharmacological action, immunopotentiation action and the like have been known. However, an insulin-mimetic action such as an anti-diabetic action or anti-obesity action has not been known so far.

Zedoary (also referred to as Murasaki-shikon), which has a nomenclature of *Curcuma zedoaria* Rosc[sic], is a plant belonging to Zingiberaceae that grows in subtropics, an its rhizome has been used as a drug from old times. As its pharmacological actions, antibacterial action, action on bile acid secretion, anti-ulcerative action, anti-tumor activity and the like have been known. However, an insulin-mimetic action such as an anti-diabetic action or anti-obesity action has not been known so far.

Crown daisy (also referred to as Kikuna, Mujinso, Koraikiku), which has a nomenclature of *Chrysanthemum coronarium,* is a plant belonging to Compositae that is native to the Southern Europe, and the Mediterranean region, and it has been known that the crown daisy richly contain nutritional components such as carotene, vitamin B2, vitamin C, calcium, and iron. As the pharmacological actions of the crown daisy, an antioxidative action, prevention of cancer or cold, an action of preventing or ameliorating anemia, an action of regulating intestines, and the like have been known. However, an insulin-mimetic action such as an anti-diabetic action or anti-obesity action has not been known so far.

Mugwort, which has a nomenclature of *Artemisia princeps* pampan, is perennial herb belonging to Compositae, and used for edible purposes in Asian countries. As the pharmacological actions of mugwort, a carcinostatic action, an action of eliminating active oxygen, a blood cleaning action, a hemostatic action and the like have been known. However, an insulin-mimetic action such as an anti-diabetic action or anti-obesity action has not been known so far.

Aloe, which has a nomenclature of *Aloe arborescens,* is perennial herb belonging to Liliaceae, of which leaves are succulent, and native to Africa, and is generally planted for appreciation purposes and medicinal purposes. Its pharmacological actions are in such a variety that the aloe is also referred to as *"isha irazu (doctors needed no more)"* and numerous pharmaceutical efficacies for constipation, deficiency in gastric acid, acute enteritis, chronic enteritis, nervous enteritis, migraine caused by cerebrovascular dilatation, gout and the like have been known. However, an insulin-mimetic action such as an anti-diabetic action or anti-obesity action has not been known so far.

Water pepper, which has a nomenclature of *Polygonum hydropiper,* is an annual herb belonging to Polygonaceae. As to its pharmacological actions, an antibacterial action, an antimycotic action, a repellent action, and the like have been known. However, an insulin-mimetic action such as an anti-diabetic action or anti-obesity action has not been known so far.

### DISCLOSURE OF INVENTION

An object of the present invention is to develop a processed product derived from a plant, having an insulin-mimetic action suitable as food materials and medicament materials, which is derived from natural products, safe and capable of being conveniently taken, to provide a medicament, food, beverage or feed using the processed product.

Summarizing the present invention, a first invention of the present invention relates to a therapeutic agent or prophylactic agent for a disease accompanying an abnormality in an amount of insulin or insulin response, characterized in that the therapeutic agent or prophylactic agent comprises as an effective ingredient a processed product derived from at least one plant selected from the group consisting of (a) a plant belonging to Tiliaceae, (b) a plant belonging to Zingiberaceae, (c) a plant belonging to Compositae, (d) a plant belonging to Liliaceae, and (e) a plant belonging to Polygonaceae.

A second invention of the present invention relates to an agent for an insulin-mimetic action, characterized in that the agent comprises as an effective ingredient a processed product derived from at least one plant selected from the group consisting of (a) to (e) mentioned above.

A third invention of the present invention relates to a food, beverage or feed for treating or preventing a disease accompanying an abnormality in an amount of insulin or insulin response, characterized in that the food, beverage or feed comprises as an effective ingredient a processed product derived from at least one plant selected from the group consisting of (a) to (e) mentioned above.

A fourth invention of the present invention relates to an agent for enhancing glucose uptake into a cell, characterized in that the agent comprises as an effective ingredient a processed product derived from at least one plant selected from the group consisting of (a) to (e) mentioned above.

A fifth invention of the present invention relates to an agent for inducing differentiation into an adipocyte, characterized in that the agent comprises as an effective ingredient a processed product derived from at least one plant selected from the group consisting of (a) to (e) mentioned above.

In the first to fifth inventions of the present invention, the plant belonging to Tiliaceae is exemplified by *Corchorus olitorius,* the plant belonging to Zingiberaceae is exemplified by *Curcuma zedoaria* Roscoe, the plant belonging to Compositae is exemplified by *Chrysanthemum coronarium* and/or *Artemisia princeps* Pampan, the plant belonging to Liliaceae is exemplified by *Aloe arborescens,* and the plant belonging to Polygonaceae is exemplified by *Polygonum hydropiper.*

### BRIEF DESCRIPTION OF THE DRAWINGS

Figure 1 is a graph showing an amount of biosynthesis of triglyceride of adipocytes that are induced to be differentiated by a water extract fraction from *Corchorus olitorius,* an ethanol extract fraction from *Corchorus olitorius,* and an ethyl acetate extract fraction from *Corchorus olitorius.*
Figure 2 is a graph showing an enhancing action by the ethanol extract fraction from *Corchorus olitorius* and the ethyl acetate extract fraction from *Corchorus olitorius* on glucose uptake.
Figure 3 is a graph showing an amount of biosynthesis of triglyceride of adipocytes that are induced to be differentiated by a water extract fraction from *Curcuma zedoaria* Roscoe, an ethanol extract fraction from *Curcuma zedoaria* Roscoe, and an ethyl acetate extract fraction from *Curcuma zedoaria* Roscoe.
Figure 4 is a graph showing an enhancing action by the ethanol extract fraction from *Curcuma zedoaria* Roscoe and the ethyl acetate extract fraction from *Curcuma zedoaria* Roscoe on glucose uptake.
Figure 5 is a graph showing an amount of biosynthesis of triglyceride of adipocytes that are induced to be differentiated by a water extract fraction from *Chrysanthemum coronarium.*
Figure 6 is a graph showing an enhancing action by the ethanol extract fraction from *Chrysanthemum coronarium* and the ethyl acetate extract fraction from *Chrysanthemum coronarium* on glucose uptake.
Figure 7 is a graph showing an amount of biosynthesis of triglyceride of adipocytes that are induced to be differentiated by an ethanol extract fraction from *Artemisia princeps* Pampan and an ethyl acetate extract fraction from *Artemisia princeps* Pampan.
Figure 8 is a graph showing an enhancing action by the ethanol extract fraction from *Curcuma zedoaria* Roscoe and the ethyl acetate extract fraction from *Curcuma zedoaria* Roscoe on glucose uptake.
Figure 9 is a graph showing an enhancing action by an ethanol extract fraction from *Polygonum hydropiper* and an ethyl acetate extract fraction from *Polygonum hydropiper* on glucose uptake.
Figure 10 is a graph showing a synergistic effect of enhancing action by the ethanol extract fraction from *Polygonum hydropiper* and insulin on glucose uptake.
Figure 11 is a graph showing an enhancing action by the ethanol extract fraction from *Polygonum hydropiper* on glucose uptake inhibited by cytochalasin B.

### BEST MODE FOR CARRYING OUT THE INVENTION

The medicament, food, beverage, feed or the like provided by the present invention comprises as an effective ingredient a processed product derived from at least one plant selected from the group consisting of the above (a) to (e). The desired effects of the present invention as described below are exhibited on the basis of the insulin-mimetic action exhibited by the effective ingredient.

In the present invention, the plant belonging to Tiliaceae is not particularly limited, as long as the plant is a plant belonging to Tiliaceae of Angiospermae, and the plant belonging to Tiliaceae is exemplified by, for example, *Corchorus olitorius, Tilia japonica, Tilia kiusiana, Tilia miqueliana, Corchorus capsularis, Corchoropsis tomentosa, Triumfetta japonica, Triumfetta procumbens,* and the like. In the present invention, *Corchorus olitorius* can be especially preferably used.

In the present invention, the plant belonging to Zingiberaceae is not particularly limited, as long as the plant is a plant belonging to Zingiberaceae of Angiospermae, and the plant belonging to Zingiberaceae is exemplified by, for example, *Curcuma zedoaria* Roscoe, *Curcuma longa* L., *Zingiber mioga, Alpinia formosana, Alpinia speciosa, Alpinia bilamellata, Zingiber officinale, Curcuma aromatica* (also referred to as Haru-ukon), *Kempferia galana,* and the like. In the present invention, *Curcuma zedoaria* Roscoe can be especially preferably used.

In the present invention, the plant belonging to Compositae is not particularly limited, as long as the plant is a plant belonging to Compositae of Angiospermae, and the plant belonging to Compositae is exemplified by, for example, *Chrysanthemum coronarium, Artemisia princeps* Pampan, *Carthamus tinctorius, Taraxacum, Helianthus annuus, Cirsium yozoense, Chrysanthemum morifolium, Arctium lappa, Petasites japonicus,* and the like. In the present invention, *Chrysanthemum coronarium* and *Artemisia princeps* Pampan can be especially preferably used.

In the present invention, the plant belonging to Liliaceae is not particularly limited, as long as the plant is a plant belonging to Liliaceae of Angiospermae, and the plant belonging to Liliaceae is exemplified by, for example, *Aloe arborescens, Allium cepa, Allium fistuiosum, Allium sativum, Tulipa gesneriana, Lilium,* and the like. In the present invention, *Aloe arborescens* can be especially preferably used.

In the present invention, the plant belonging to Polygonaceae is not particularly limited, as long as the plant is a plant belonging to Polygonaceae of Angiospermae, and the plant belonging to Polygonaceae is exemplified by, for example, *Polygonum hydropiper, Rumex acetosa, Rumex japonicus, Rumex madaio, Polygonum aviculare, Persicaria pilosa, Polygonum longiestum, Polygonum hydropiper* var. *fastigiatum, Persicaria hydropiper* var. *maximowiczii, Polygonum flaccidum, Polygonum yokusaianum, Persicaria erecto-minor, Polygonum trigonocarpum, Polygonum taquetti, Polygonum paludicola, Persicaria viscofera, Polygonum persicaria, Polygonum lapathifolium, Polygonum tinctorium, Polygonum conspicuum, Polygonum viscosum, Polygonum amphibium, Polygonum nepalense, Fallopia dumetora, Polygonum chinense, Polygonum cuspidatum, Fagopyrum esculentum,* and the like. In the present invention, *Polygonum hydropiper* can be especially preferably used.

In addition, the plant usable in the present invention is not particularly limited, and fruit, seed, seed coat, flower, leaf, stem, root, rhizome and/or whole plant can be used as it is.

The processed product derived from the various plants usable as the effective ingredient of the present invention is not particularly limited, as long as the processed product is obtained by subjecting a raw material plant to some sort of processing, wherein the processed product has an insulin-mimetic action. The processed product refers to, for example, an extract, a powder, a squeezed juice, a pulverized product, a chemically processed product, or an enzymatically processed product, and is especially preferably exemplified by an extract, a powder and a squeezed juice.

In the present invention, the insulin-mimetic action is not particularly limited as long as at least one of the physiological activities possessed by insulin is exhibited. For example, the insulin-mimetic action is exemplified by metabolic regulatory actions such as enhancement of uptake of a sugar or an amino acid in a cell, and synthesis and degradation inhibition of glycogen or protein. Also, the presence or absence of the insulin-mimetic action can be conveniently determined in accordance with the method described in Example 2 or 3 set forth below.

In the present invention, the extract refers to a substance itself obtained through the process of subjecting a raw material plant to an extraction procedure with an extraction solvent. The extraction can be carried out as follows by a known extraction method. For example, the raw material is powdered or cut into thin pieces, and thereafter extracted in a batch process or continuous process using a solvent. The extraction solvent used upon obtaining an extract is not particularly limited, and includes, for example, hydrophilic or lipophilic solvents such as water, chloroform, alcohols such as ethanol, methanol and isopropyl alcohol, ketones such as acetone and methyl ethyl ketone, methyl acetate, and ethyl acetate, which can be used alone or properly as a mixed solution as desired. The amount of the extraction solvent may be appropriately determined. Usually, the extraction solvent may be used in an amount of the weight of the raw material plant in the form as it is upon use (for example, if the raw material plant is a raw plant, the weight of the raw plant), preferably in an amount of from 0.1-to 100-folds by weight of the raw materials. The extraction temperature may be also appropriately determined according to its purposes. In the case of the water extraction, usually, the extraction temperature is in the range of preferably from 4° to 130°C, more preferably from 25° to 100°C. Alternatively, in the case where ethanol is contained in the solvent, the extraction temperature is preferably within the range of from 4° to 60°C. The extraction time may be also determined in consideration of extraction efficiency. It is usually preferable that the raw materials, the extraction solvent and the extraction temperature are set so that the extraction time is within the range of preferably from several seconds to several days, more preferably 5 minutes to 24 hours. The extraction procedure may be carried out, for example, while stirring or allowing the mixture to stand. Also, the extraction procedures may be repeated several times as desired. By the above procedures, an extract derived from the various plants usable in the present invention (hereinafter referred to as the extract of the present invention in some cases) can be obtained. The extract is subjected to such a process as filtration, centrifugation, concentration, ultrafiltration or molecular sieving as desired, whereby an extract in which a substance of interest having an insulin-mimetic action is concentrated can be prepared. The insulin-mimetic action of the extract or concentrated extract can be conveniently determined in accordance with the method described in Example 2 or 3 set forth below. Alternatively, the various plants usable in the present invention may be processed in the form like tea-leaves by a known method, and an extract obtained from the tea-leaf-like products can be used as the extract of the present invention as long as the extract has an insulin-mimetic action. Also, two or more kinds of the above extracts can be contained to be used. In the present invention, two or more kinds of extracts obtained by different extraction methods from the raw material plants can be contained to be used.

In addition, in the present invention, a fraction obtained by fractionating the extract of the present invention by a known method, or a fraction obtained by repeating the fractionation procedures a plural times is also encompassed in the extract of the present invention. The above-mentioned fractionation means include extraction, separation by precipitation, column chromatography, thin-layer chromatography, and the like. The substance having an insulin-mimetic action can also be isolated by further proceeding the purification of the resulting fraction using the insulin-mimetic action as an index.

Alternatively, a processed product derived from various plants usable in the present invention other than the extract of the present invention is exemplified by, for example, a powder derived from various plants usable in the present invention (which may be hereinafter referred to as the powder of the present invention in some cases). As a method for preparing a powder of the present invention, for example, a plant is dried and powdered with a powdering machine, whereby a powder derived from a plant can be obtained. In addition, the powder may be obtained by lyophilization.

In addition, the squeezed juice derived from various plants usable in the present invention can be used as a processed product of the present invention. The method for preparing the squeezed juice is not particularly limited, as long as the method is a known method of squeezing a plant. For example, the method includes a method using a squeezer of a screw-type, a gear-type, a cutter-type or the like, or a juicer. Also, the raw materials may be cut into thin pieces or mashed as a pre-processing, and thereafter squeezed with the above-mentioned juicer or cloth or the like, whereby a squeezed juice can be obtained.

A pulverized product refers to one prepared by pulverizing the raw material plant, and its tissue piece is generally larger than the powder. For example, the pulverized product can be prepared by using a pulverizer. Also, the chemically processed product is not particularly limited, and refers to a product obtained by subjecting the raw material plant to an acid processing, an alkali processing, an oxidation processing, a reducing processing or the like. The chemically processed product can be prepared, for example, by immersing the raw material plant in an aqueous solution containing an inorganic acid or organic acid, such as hydrochloric acid, sulfuric acid, nitric acid, citric acid or acetic acid, or an inorganic base or organic base, such as sodium hydroxide, potassium hydroxide or ammonia. The chemically processed product includes all those derived from plants subjected to chemical processing as mentioned above. The enzymatically processed product refers, for example, to an enzymatically processed product processed with pectinase, cellulase, xylanase, amylase, mannanase, or glucosidase, an enzymatic reaction product by a microorganism (for example, a fermented product) or the like. The enzymatically processed product can be prepared, for example, by allowing the above-mentioned enzyme to act on the raw material plant in an appropriate buffer. The enzymatically processed product includes all those derived from plants subjected to the enzymatic processing as mentioned above. Further, the processed product derived from the plant of the present invention encompasses, for example, juice obtained by cutting the stem of the raw material plant and obtaining juice from its cross section.

In the present invention, the shape of the processed product of the present invention is not particularly limited as long as the processed product can exhibit an insulin-mimetic action when the processed product is used as an effective ingredient in each of the embodiments of the present invention, and the processed product may take any form of powder, solid or liquid. In addition, the processed product can be used in the form of a granular solid prepared by granulating the processed product by a known process. The granulation process is not particularly limited, and is exemplified by tumbling granulation, agitation granulation, fluidizing bed granulation, airflow granulation, extruding granulation, compression molding granulation, disintegration granulation, spray granulation, spray-dry granulation or the like. In addition, the powdery processed product can be used as the processed product derived from the various plants of the present invention in the form of a liquid prepared by dissolving the powdery processed product in a liquid, for example, water, an alcohol or the like.

In addition, the present invention provides a food, beverage or feed containing the processed product of the present invention in a high concentration or at high purity. The food, beverage or feed contains a substance having an insulin-mimetic action in a high concentration and/or at high purity, as compared to that of a conventional food, beverage or feed.

In the present invention, the processed product of the present invention may be referred to as the effective ingredient of the present invention, and the therapeutic agent or prophylactic agent for a disease accompanying an abnormality in an amount of insulin or insulin response, wherein the therapeutic agent or prophylactic agent comprises the effective ingredient of the present invention may be referred to as the therapeutic agent or prophylactic agent of the present invention. Also, in the case of the medicament of the present invention, the medicament may encompass an agent for an insulin-mimetic action in addition to the therapeutic agent and the prophylactic agent.

No toxicity is especially found in the effective ingredient of the present invention as mentioned later. Also, there is no risk of the onset of side effects. For these reasons, the disease can be safely and appropriately treated or prevented. Therefore, the therapeutic agent, the prophylactic agent, the food, the beverage or the feed, each comprising the effective ingredient, is effective for treating or preventing a disease accompanying an abnormality in an amount of insulin or insulin response.

In the present invention, the disease accompanying an abnormality in an amount of insulin or insulin response includes diseases characterized by a factor selected from change in insulin level in blood, change in activity level of insulin or an insulin receptor, aberrance in downstream signal of an insulin receptor and a combination thereof. The disease is exemplified by, for example, diabetes, obesity, hypertension, arteriosclerosis, cocaine withdrawal symptoms, static cardiac incompetence, amnesia, cardiovascular spasm, cerebral angiospasm, chromaffinoma, ganglioneuroblastoma, Huntington's disease, and hyperlipemia. The diabetes may be exemplified by both type I diabetes and type II diabetes. In addition, the type II diabetes encompasses a disease of which causation is insulin resistance for which a therapeutic effect is not found even when insulin or an agent for enhancing insulin secretion is administered.

In addition, the disease accompanying an abnormality in an amount of insulin or insulin response is more likely to lead to deficiency in the amount of insulin production, or deficiency in the action of insulin caused by insulin resistance in the onset stage of the disease. Since the effective ingredient of the present invention can suppress the onset of the disease accompanying an abnormality in an amount of insulin or insulin response by showing an insulin-mimetic action, the prophylactic effects of the disease can be also expected.

In the state of insulin resistance, a signal from an insulin receptor by insulin is inhibited, so that diversified functions possessed by insulin are not exhibited, whereby generating various dysbolisms. The effective ingredient used in the present invention can exhibit an insulin-mimetic effect also on a symptom of insulin resistance. Specifically, by using the prophylactic agent or therapeutic agent of the present invention, a therapeutic or prophylactic effect can be exhibited also for a disease caused by insulin resistance, for example, type II diabetes for which a therapeutic effect is not seen even when insulin or an agent for enhancing insulin secretion were administered. In addition, the effective ingredient of the present invention can also exhibit the effect of lowering the amount of insulin in blood. In other words, the medicament of the present invention can be also used as a therapeutic agent or prophylactic agent for a disease requiring lowering of the amount of insulin for treatment or prevention. The disease is not particularly limited, and is exemplified by hyperinsulinemia, Alzheimer's disease and the like. In addition, since reports have been made that the stimulation via an insulin receptor and the effect of extended longevity are closely related (*Science,* vol. 299, P572-574 (2003); *Nature,* vol. 424, P277-284 (2003)), the medicament of the present invention can also be used as an agent for anti-aging.

There have been known that insulin enhances induction of differentiation of preadipocytes into adipocytes, and that glucose uptake takes place in matured adipocytes thereby accumulating triglyceride in the adipocytes *(J. Biol. Chem.,* Vol. 253, No. 20, P7570-7578 (1978)). Specifically, utilizing this method, an insulin-mimetic action of a test substance can be determined by administering a test substance in place of insulin, and determining differentiation into an adipocyte and the amount of triglyceride in the adipocytes.

In addition, there have been known that insulin has an enhancing action on glucose uptake into a cell, and that glucose uptake into the cell is enhanced by the action of insulin in a matured adipocyte *(J. Biol. Chem.,* Vol. 253, No. 20, P7579-7583 (1978)). Specifically, utilizing this method, an insulin-mimetic action of a test substance can be determined by administering a test substance in place of insulin, and determining the amount of glucose uptake into a matured adipocyte.

The therapeutic agent or prophylactic agent of the present invention includes ones formed into a preparation by combining the above-mentioned effective ingredient according to the present invention with a known pharmaceutical carrier. Also, as the therapeutic agent or prophylactic agent of the present invention, the above-mentioned effective ingredient can be combined with other components which can be used for the same applications as those of the effective ingredients, for example, an insulin preparation, an agent for enhancing insulin secretion, an agent for improving insulin resistance, an agent for ameliorating postprandial hyperglycemia, an agent for an insulin-mimetic action or the like which is known in the art. The therapeutic agent or prophylactic agent can also be combined together with a processed product derived from a plant belonging to Umbelliferae having an insulin-mimetic action described in WO 04/014407.

The therapeutic agent or prophylactic agent of the present invention is usually manufactured by combining the above-mentioned effective ingredient with a pharmacologically acceptable liquid or solid carrier. A solvent, a dispersant, an emulsifier, a buffer, a stabilizer, an excipient, a binder, a disintegrant, a lubricant, or the like can be optionally added thereto, to form a solid agent such as a tablet, a granule, a powder, an epipastic, and a capsule, or a liquid agent such as a common liquid agent, a suspension agent or an emulsion agent. In addition, there can be also formed into a dry product which can be liquefied by adding an appropriate carrier before use, or also into an external preparation.

The pharmaceutical carrier can be selected depending upon the administration form and preparation form of the therapeutic agent or prophylactic agent. In the case of an orally administered preparation comprising a solid composition, the preparation can be produced in the form of a tablet, a pill, a capsule, a powder, a fine powder, a granule or the like, and there can be utilized, for example, starch, lactose, saccharose, mannitol, carboxymethyl cellulose, cornstarch, an inorganic salt or the like. In addition, upon preparation of the orally administered preparation, a binder, a disintegrant, a surfactant, a lubricant, a fluidity accelerator, a corrective, a colorant, a flavor, and the like can be further combined therewith. In the case of forming into a tablet or a pill, for example, the tablet or pill may be covered with a sugar-coating made of sucrose, gelatin or hydroxypropyl cellulose, or with a film made of a substance soluble in the stomach or intestine as desired. In the case of an orally administered preparation comprising a liquid composition, the preparation can be prepared in the form of a pharmaceutically acceptable emulsion, solution, suspension, syrup, or the like. In this case, for example, purified water, ethanol or the like is utilized as a carrier. Furthermore, an auxiliary agent such as a wetting agent or a suspending agent, a sweetener, a flavor, an antiseptic, or the like may be added as desired.

On the other hand, in the case of a parenterally administered preparation, the preparation can be prepared by dissolving or suspending the above-mentioned effective ingredient of the present invention in a diluent such as distilled water for injection, physiological saline, an aqueous solution of glucose, vegetable oil for injection, sesame oil, peanut oil, soybean oil, corn oil, propylene glycol or polyethylene glycol, in accordance with a conventional method, and adding a microbicide, a stabilizer, a tonicity agent, a soothing agent, or the like if needed. It is also possible to produce a solid composition and dissolve the composition in sterile water or a sterile solvent for injection before use.

The external preparation includes solid, semi-solid or liquid preparations for percutaneous administration or transmucosal (intraoral or intranasal) administration. The external preparation also includes suppositories and the like. For example, the external preparation may be prepared as liquid preparations including emulsions, suspensions such as lotions, external tinctures, and liquid agents for transmucosal administration; ointments such as oily ointments and hydrophilic ointments; patches for percutaneous administration or transmucosal administration such as films, tapes and poultices; and the like.

Each of the above-mentioned various preparations can be appropriately produced in accordance with conventional methods by utilizing known pharmaceutical carriers and the like. Also, the content of the effective ingredient in the preparation is not particularly limited, as long as the content is preferably in an amount so that the effective ingredient can be administered within the dose range described below in consideration of administration form, administration method and the like of the preparation. The content of the effective ingredient in the medicament of the present invention is usually from 1 to 100% by weight or so.

The therapeutic agent or prophylactic agent of the present invention is administered via an administration route appropriate for each of the preparation form. The administration method is also not limited to specific one. The agent can be administered internally, externally (or topically) and by injection. The injection can be administered, for example, intravenously, intramuscularly, subcutaneously, intracutaneously, or the like. As to an external preparation, for example, a suppository may be administered according to its proper administration method.

The dose of the therapeutic agent or prophylactic agent of the present invention is changeable and properly set depending upon its preparation form, administration method, purpose of use, and age, body weight, symptom or the like of a patient to which the therapeutic agent or prophylactic agent is administered. Generally, the dose of the agent, in terms of the dose of the above-mentioned effective ingredient contained in the preparation, is preferably from 0.1 µg to 10 g/kg weight, more preferably from 1 µg to 5 g/kg weight, and even more preferably from 10 µg to 1 g/kg weight, per day for adult. As a matter of course, the dose varies depending upon various conditions, so that an amount smaller than the dose mentioned above may be sufficient, or an amount exceeding the dose range may be required. Administration may be carried out once or in several divided portions in a day within the desired dose range. The administration period may be arbitrarily determined. Also, the therapeutic agent or prophylactic agent of the present invention can be orally administered as it is, or the agent can be added to any foodstuffs to be taken on a daily basis.

In addition, the present invention can provide an agent for an insulin-mimetic action comprising the above-mentioned effective ingredient. The agent for an insulin-mimetic action may be the above-mentioned effective ingredient itself, or a composition comprising the above-mentioned effective ingredient. The agent for an insulin-mimetic action may be prepared by, for example, combining the above-mentioned effective ingredient with other component, for example, an insulin preparation, an agent for enhancing insulin secretion, an agent for improving insulin resistance, an agent for ameliorating postprandial hyperglycemia, an agent for an insulin-mimetic action or the like which is known in the art which can be used for the same application as that of the effective ingredient, and forming into a form of reagent usually used according to the above-mentioned process for preparing the therapeutic agent or prophylactic agent. In addition, the agent for an insulin-mimetic action can be combined together with a processed product derived from a plant belonging to Umbelliferae having an insulin-mimetic action described in WO 04/014407. The content of the above-mentioned effective ingredient in the agent for an insulin-mimetic action is not particularly limited, as long as the content is in an amount so that the desired effects of the present invention can be exhibited in consideration of administration method, purpose of use or the like of the agent for an insulin-mimetic action. The content of the effective ingredient in the agent for an insulin-mimetic action of the present invention is usually from 1 to 100% by weight or so. Also, the amount of the agent for an insulin-mimetic action used is not particularly limited, as long as the desired effects of the present invention can be exhibited. Especially in the case where the agent for an insulin-mimetic action is administered to a living body, the agent for an insulin-mimetic action may be preferably used in an amount so that the effective ingredient can be administered within the dose range of the effective ingredient for the above-mentioned therapeutic agent or prophylactic agent. The agent for an insulin-mimetic action is useful in treating or preventing a disease accompanying an abnormality in an amount of insulin or insulin response. In addition, the agent for an insulin-mimetic action is useful in screening of drugs for diseases accompanying an abnormality in an amount of insulin or insulin response. Furthermore, the agent for an insulin-mimetic action is useful in studies on mechanisms of an action of insulin on cells, or functional studies relating to physical changes in the cells. In addition, the agent for an insulin-mimetic action can be used by adding the agent in place of or together with serum or insulin preparation to a medium for culturing a cell, a tissue, or an organ. The medium is very useful as a medium for culturing a cell, a tissue or an organ that has reduced level of, or contains no serum or insulin preparation.

In addition, administration of the agent for an insulin-mimetic action of the present invention to human can be expected to lower the amount of insulin in blood. In other words, the agent for an insulin-mimetic action of the present invention can also be used as a therapeutic or prophylactic agent for a disease requiring the lowering of the amount of insulin for the treatment or prevention. The disease is not particularly limited, and is exemplified by hyperinsulinemia, Alzheimer's disease and the like. In addition, since reports have been made that the stimulation via an insulin receptor and the effect of extended longevity are closely related *(Science,* vol. 299, P572-574 (2003); *Nature,* vol. 424, P277-284 (2003)), the agent for an insulin-mimetic action of the present invention can also be used as an agent for anti-aging.

No toxicity is especially found in the effective ingredient of the present invention as described later. Also, there is no risk of the onset of side effects. For these reasons, the insulin-mimetic action can be safely and appropriately exhibited. Therefore, the medicament, food, beverage or feed of the present invention comprising the effective ingredient is effective for treating or preventing a disease accompanying an abnormality in an amount of insulin or insulin response.

In addition, the present invention provides a food, beverage or feed for treating or preventing a disease accompanying an abnormality in an amount of insulin or insulin response, wherein the food, beverage or feed comprises the above-mentioned effective ingredient. Since the food, beverage or feed of the present invention has an insulin-mimetic action, the food, beverage or feed is very useful in amelioration of symptoms or prevention of a disease accompanying an abnormality in an amount of insulin or insulin response. Furthermore, the food or beverage of the present invention is a food or beverage for lowering blood sugar level, having the action of lowering a blood sugar level, so that the food or beverage is useful as a functional food or beverage effective for an individual who cares about his/her blood sugar level or an individual who cares about his/her body fat.

In the food, beverage or feed of the present invention, the effective ingredient of the present invention can be combined with another substance which is known to have an anti-diabetic action, for example, a substance having an insulin-mimetic action, a substance having enhancing action on insulin secretion, a substance having an action of improving insulin resistance, or a substance having an action of ameliorating postprandial hyperglycemia, which is commonly known. The food, beverage or feed can also be combined with, for example, hardly digestible dextrin or the like. In addition, the food, beverage or feed can be combined together with a processed product derived from a plant belonging to Umbelliferae having an insulin-mimetic action described in WO 04/014407.

The term "comprising" in the food, beverage or feed of the present invention encompasses the meanings of containing(ed), adding(ed) and/or diluting(ed). As used herein, the term "containing(ed)" refers to an embodiment of containing the effective ingredient used in the present invention in the food, beverage or feed; the term "adding(ed)" refers to an embodiment of adding the effective ingredient used in the present invention to a raw material for the food, beverage or feed; and the term "diluting(ed)" refers to an embodiment of adding a raw material for the food, beverage or feed to the effective ingredient used in the present invention.

The process for preparing the food, beverage or feed of the present invention is not particularly limited. For example, combination, cooking, processing, and the like can be carried out in accordance with those generally employed for foods, beverages or feeds, and the food, beverage or feed of the present invention can be prepared by the general methods for preparing a food, beverage or feed, as long as the resulting food, beverage or feed contains the above-mentioned effective ingredient of the present invention, wherein the effective ingredient has an insulin-mimetic action.

The food or beverage of the present invention is not particularly limited. The food or beverage includes, for example, processed agricultural and forest products, processed livestock products, processed marine products and the like, including processed grain products such as processed wheat products, processed starch products, processed premix products, noodles, macaronis, bread, bean jam, buckwheat noodles, wheat-gluten bread, rice noodles, *fen-tiao,* and packed rice cake; processed fat and oil products such as plastic fat and oil, tempura oil, salad oil, mayonnaise, and dressing; processed soybean products such as tofu products, soybean paste *(miso),* and fermented soybeans; processed meat products such as ham, bacon, pressed ham, and sausage; marine products such as frozen ground fish, steamed fish paste, tubular roll of steamed fish paste, cake of ground fish, deep-fried patty of fish paste, fish ball, fascia and tendon, fish meat ham, sausage, dried bonito, products of processed fish egg, canned marine products, and preserved food boiled down in soy sauce *(tsukudani);* milk products such as raw material milk, cream, yogurt, butter, cheese, condensed milk, powder milk, and ice cream; processed vegetable and fruit products such as paste, jam, pickled vegetables, fruit beverages, vegetable beverages, and mixed beverages; confectionaries such as chocolates, biscuits, sweet bun, cake, rice cake snacks and rice snacks; alcohol beverages such as *sake,* Chinese liquor, wine, whiskey, Japanese distilled liquor *(shochu),* vodka, brandy, gin, rum, beer, refreshing alcoholic beverages, fruit liquor, and liqueur; luxury drinks such as green tea, tea, oolong tea, coffee, refreshing beverages and lactic acid beverages; seasonings such as soy sauce, sauce, vinegar, and sweet rice wine; canned, bottled or pouched foods such as rice topped with cooked beef and vegetable, rice boiled together with meat and vegetables in a small pot, steamed rice with red beans, curry roux and rice, and other precooked foods; semi-dry or concentrated foods such as liver pastes and other spreads, soups for buckwheat noodles or wheat noodles, and concentrated soups; dry foods such as instant noodles, instant curry roux, instant coffee, powder juice, powder soup, instant soybean paste *(miso)* soup, precooked foods, precooked beverages, and precooked soup; frozen foods such as *sukiyaki,* pot-steamed hotchpotch, split and grilled eel, hamburger steak, *shao-mai,* dumpling stuffed with minced pork, various sticks, and fruit cocktails; solid foods; liquid foods such as soups; spices; and the like, in which each of the foods and beverages comprises the above-mentioned effective ingredient according to the present invention.

In the food or beverage of the present invention, the above-mentioned effective ingredient is contained, added and/or diluted, alone or in plurality, and its shape is not particularly limited, as long as the effective ingredient is contained in an amount necessary for exhibiting an insulin-mimetic action. The shape includes those which can be taken orally such as tablets, granules and capsules. In addition, the food of the present invention may encompass a processed product of a raw material plant as it is, or a product obtained by properly mixing the processed product with an appropriate emulsifying agent or the like, and solidifying the mixture.

In addition, as to the beverage of the present invention, there can be prepared into healthcare drink by mixing the effective ingredient of the present invention with a squeezed juice of a plant other than those plants usable in the present invention, for example, a vegetable, a fruit or the like, or squeezing the plant together with the plant usable in the present invention. For example, the healthcare drink having an insulin-mimetic action can be prepared by diluting a squeezed juice of various plants with water, or mixing the squeezed juice with a squeezed juice of *Angelica keiskei* koidz., parsley, celery, licorice, carrot, *Brassica Rapa* var. *pervidis (komatsuna),* turnip, pak-choi, tomato, mandarin orange, lemon, grapefruit, kiwi, spinach, radish, Japanese radish *(daikon),* celery cabbage, cabbage, Butter head lettuce, lettuce, Chinese chive, okra, green pepper, cucumber, kidney beans, green soybeans, common pea, Indian corn, *Rocket,* arugula loquat, *Citrus natsudaidai, amanatsu,* or the like, cow's milk, soybean milk or the like.

The content of the above-mentioned effective ingredient in the food or beverage of the present invention is not particularly limited, and the content can be appropriately selected from the viewpoints of sensory aspect and exhibition of activity. The content of the effective ingredient is, for example, preferably 0.1% by weight or more, more preferably from 0.5 to 95% by weight, even more preferably from 1 to 90% by weight, of the food. The content is, for example, preferably 0.1% by weight or more, more preferably from 0.5 to 95% by weight, even more preferably from 1 to 90% by weight, of the beverage. Also, the food or beverage of the present invention may be taken so that the effective ingredient contained therein is taken preferably in an amount of, for example, from 0.1 µg to 10 g/kg weight, preferably from 1 µg to 5 g/kg weight, and more preferably from 10 µg to 1 g/kg weight, per day for adult.

In addition, the present invention provides a feed for an organism having an insulin-mimetic action, in which the feed comprises the above-mentioned effective ingredient, in other words, the effective ingredient is contained, added and/or diluted. In still another embodiment, the present invention also provides a method of feeding an organism, characterized by administering the above-mentioned effective ingredient to the organism. In still yet another embodiment, the present invention provides an organism-feeding agent characterized in that the organism-feeding agent comprises the above-mentioned effective ingredient.

In these inventions, the organisms are, for example, cultured or bred animals, pet animals, and the like. The cultured or bred animal is exemplified by livestock, laboratory animals, poultry, pisces, crustacea or shellfish. The feed is exemplified by a feed for sustenance of and/or amelioration in physical conditions. The organism-feeding agent is exemplified by immersion agents, feed additives, and beverage additives.

According to these inventions, the same effects can be expected to be exhibited as those of the above-mentioned therapeutic agent or prophylactic agent of the present invention, on the basis of the insulin-mimetic action of the above-mentioned effective ingredient used in the present invention, in the organism exemplified above to which these are applied. In other words, the feed of the present invention can exhibit an effect for treating or preventing a disease accompanying an abnormality in an amount of insulin or insulin response in the organism.

The above-mentioned effective ingredient used in the present invention is usually administered in an amount of preferably from 0.01 to 2000 mg per day per 1 kg of the body weight of a subject organism. The administration can be carried out, for example, by previously adding and mixing the effective ingredient in a raw material for an artificially formulated feed to be given to a subject organism, or mixing the effective ingredient with a powder raw material for an artificially formulated feed, and thereafter further adding and mixing the mixture with other raw materials. The content of the above-mentioned effective ingredient in the feed is not particularly limited. The content can be appropriately set in accordance with the purposes, and the content is preferably in a ratio of from 0.001 to 15% by weight. The content of the effective ingredient of the present invention in the organism-feeding agent may be adjusted to the same level as the feed.

The process for preparing the feed of the present invention is not particularly limited, and its composition may be set in accordance with a general feed, as long as the above-mentioned effective ingredient according to the present invention having an insulin-mimetic action is contained in the feed prepared. The organism-feeding agent can also be prepared in the same manner.

The organism to which the present invention can be applied is not limited. The cultured or bred animals include livestock such as *Equus, Bos, Porcus, Ovis, Capra, Camelus,* and *Lama;* laboratory animals such as mice, rats, guinea pigs, and rabbits; poultry such as *Chrysolophus,* ducks, *Meleagris,* and *Struthioniformes;* and the pet animals include dogs, cats, and the like, so that the feed can be widely applied.

In the present invention, by, for example, allowing a subject organism to take the feed comprising the above-mentioned effective ingredient used in the present invention having an insulin-mimetic action, or immersing a subject organism into a solution containing the above-mentioned effective ingredient used in the present invention having an insulin-mimetic action (for example, prepared by dissolving the above immersing agent in water), the physical conditions of the livestock, laboratory animals, poultry, pet animals or the like can be well sustained or ameliorated. These embodiments are one embodiment of the feeding method of an organism of the present invention.

In addition, the present invention can provide an agent for enhancing glucose uptake into a cell comprising the above-mentioned effective ingredient. The agent for enhancing glucose uptake may be the above-mentioned effective ingredient itself, or a composition comprising the above-mentioned effective ingredient. The agent for enhancing glucose uptake may be prepared by, for example, combining the above-mentioned effective ingredient with another component, for example, an insulin preparation, an agent for enhancing insulin secretion, an agent for improving insulin resistance, an agent for ameliorating postprandial hyperglycemia, an agent for an insulin-mimetic action or the like that is commonly known, which can be used for the same application as that of the effective ingredient, and forming into a form of reagent usually used according to the above-mentioned process for preparing the therapeutic agent or prophylactic agent. In addition, the agent for enhancing glucose uptake can be combined together with a processed product derived from a plant belonging to Umbelliferae having an enhancing action on glucose uptake into a cell described in WO 04/014407. The content of the above-mentioned effective ingredient in the agent for enhancing glucose uptake is not particularly limited, as long as the content is in an amount so that the desired effects of the present invention can be exhibited in consideration of administration method, purpose of use or the like of the agent for enhancing glucose uptake. The content of the effective ingredient in the agent for enhancing glucose uptake of the present invention is usually from 1 to 100% by weight or so. Also, the amount of the agent for enhancing glucose uptake used is not particularly limited, as long as the desired effects of the present invention can be exhibited. Especially in the case where the agent for enhancing glucose uptake is used by administering to a living body, the agent for enhancing glucose uptake may be used preferably in an amount so that the effective ingredient can be administered within the dose range of the effective ingredient for the above-mentioned therapeutic agent or prophylactic agent. The agent for enhancing glucose uptake is useful in treating or preventing a disease requiring an enhancing action on glucose uptake into a cell for the treatment or prevention. The disease is exemplified by, for example, the above-mentioned disease requiring an insulin-mimetic action for the treatment or prevention, as well as cardiac diseases, especially cardiac infarction and post-ischemic injury of the heart, and the like. In addition, since the agent for enhancing glucose uptake enhances glucose uptake by a cell, when the action is exhibited in a muscle cell, an enhancing action on muscles or an action on recovery from fatigue can be induced. Also, the agent for enhancing glucose uptake can be used for the manufacture of a food, beverage or feed for treating or preventing these diseases. The food, beverage, or feed can be used according to the above-mentioned food, beverage or feed of the present invention. In addition, the agent for enhancing glucose uptake is also useful in screening of drugs for the above-mentioned diseases requiring an enhancing action on glucose uptake into a cell for the treatment or prevention. Furthermore, the agent for enhancing glucose uptake is useful in studies on mechanisms of action on glucose uptake by the cell, or functional studies on physical changes in the cell and the like.

In addition, the present invention can provide an agent for inducing differentiation into an adipocyte comprising the above-mentioned effective ingredient. The precursor cell that can be induced to differentiate into an adipocyte by the agent for inducing differentiation is not particularly limited, as long as the cell is capable of differentiating into adipocytes. The precursor cell includes, for example, preadipocyte, fibroblast, mesenchymal stem cell and the like. The agent for inducing differentiation may be the above-mentioned effective ingredient itself, or a composition comprising the above-mentioned effective ingredient. The agent for inducing differentiation may be prepared by, for example, combining the above-mentioned effective ingredient with other component, for example, an insulin preparation, an agent for enhancing insulin secretion, an agent for improving insulin resistance, an agent for ameliorating postprandial hyperglycemia, an agent for an insulin-mimetic action or the like which is commonly known, which can be used for the same application as that of the effective ingredient, and forming into a form of reagent usually used according to the above-mentioned process for preparing the therapeutic agent or prophylactic agent. In addition, the agent for inducing differentiation can be combined together with a processed product derived from a plant belonging to Umbelliferae having an action of inducing differentiation into an adipocyte described in WO 04/014407. The content of the above-mentioned effective ingredient in the agent for inducing differentiation is not particularly limited, as long as the content is in an amount so that the desired effects of the present invention can be exhibited in consideration of administration method, purpose of use or the like of the agent for inducing differentiation. The content of the effective ingredient in the agent for inducing differentiation of the present invention is usually from 1 to 100% by weight or so. Also, the amount of the agent for inducing differentiation used is not particularly limited, as long as the desired effects of the present invention can be exhibited. Especially in the case where the agent for inducing differentiation is administered to a living body, the agent for inducing differentiation may be used preferably in an amount so that the effective ingredient can be administered within the dose range of the effective ingredient for the above-mentioned therapeutic agent or prophylactic agent. The agent for inducing differentiation is useful in treating or preventing a disease requiring an action of inducing differentiation into an adipocyte for the treatment or prevention. The disease is exemplified by, for example, the above-mentioned disease requiring an insulin-mimetic action for the treatment or prevention, as well as gout, fatty liver, cholecystolithiasis, menoxenia, infertility, and the like. The agent for inducing differentiation can be used for the manufacture of a food, beverage or feed for treating or preventing these diseases. The food, beverage, or feed can be used according to the above-mentioned food, beverage or feed. In addition, the agent for inducing differentiation is also useful in screening of drugs for diseases requiring an action of inducing differentiation into an adipocyte for the treatment or prevention. Furthermore, the agent for inducing differentiation is useful in studies on mechanisms of action of inducing differentiation into an adipocyte, or functional studies on physical changes in the cell and the like.

No toxicity is found even when the above-mentioned effective ingredient used in the present invention is administered in an effective dose for the exhibition of its action. For example, in the case of oral administration, no cases of deaths are found even when an ethanol extract of *Corchorus olitorius, Aloe arborescens, Curcuma zedoaria* Roscoe, *Chrysanthemum coronarium, Polygonum hydropiper* or *Artemisia princeps* Pampan is administered to a mouse at 1g/kg weight in a single dose. In addition, no cases of deaths are found even when the above-mentioned effective ingredient is orally administered to a rat at 1 g/kg weight in a single dose.

### EXAMPLES

The present invention will be described more concretely hereinbelow by means of the examples, but the present invention is by no means limited to these descriptions. Unless specified otherwise, % in these examples means % by volume.

### Example 1 Preparation of Extract Fraction from Corchorus olitorius

Forty milliliters of distilled water, ethanol or ethyl acetate was added to 2 g each of a dry powder of *Corchorus olitorius,* and extraction was carried out at room temperature for 30 minutes. The mixture was separated into an extract and a residue by centrifugation. Next, the extraction procedures with 30 ml of the same solvent were repeated twice for the residue. Here, the extraction with water was carried out at 60°C, and the extraction with ethanol and the extraction with ethyl acetate were carried out at room temperature. Each of these extracts obtained was combined, and the combined extract was concentrated with a rotary evaporator. Finally, a water extract was dissolved in 20 ml of distilled water, to give a water extract fraction from *Corchorus olitorius.* An ethanol extract was dissolved in 5 ml of dimethyl sulfoxide, to give an ethanol extract fraction from *Corchorus olitorius.* An ethyl acetate extract was dissolved in 5 ml of dimethyl sulfoxide, to give an ethyl acetate extract fraction from *Corchorus olitorius.*

### Example 2 Induction of Differentiation into Adipocytes by Extract Fractions from Corchorus olitorius

### (1) Induction of Differentiation into Adipocytes

The induction of differentiation into adipocytes was carried out by partially modifying the above-mentioned method of Rubin C. S. et al. (*J. Biol. Chem.,* Vol. 253, No. 20, P7570-7578 (1978)). Preadipocyte cell line 3T3-L1 (ATCC CCL-92.1) was suspended in a 10% fetal bovine serum (manufactured by GIBCO)-containing Dulbecco's modified Eagle's medium (manufactured by Sigma, D6046) containing 200 µM ascorbic acid (hereinafter referred to as A-D-MEM medium) so as to have a density of 4 × 10³ cells/ml, and the suspension was put in each well of a 12-well microtiter plate in an amount of 2 ml per well. The cells were cultured at 37°C for 7 days in the presence of 5% carbon dioxide gas. The medium was exchanged with the same medium on the second day and the fourth day. On the seventh day, the medium was exchanged with A-D-MEM medium containing 0.25 µM dexamethasone. Thereafter, the aqueous solution of the water extract fraction from *Corchorus olitorius* prepared in Example 1 was added thereto so as to have a final concentration of 0.1%, or the dimethyl sulfoxide solution of the ethanol extract fraction from *Corchorus olitorius* prepared in Example 1 was added thereto so as to have a final concentration of 0.067%, or the dimethyl sulfoxide solution of the ethyl acetate extract fraction from *Corchorus olitorius* prepared in Example 1 was added thereto so as to have a final concentration of 0.067%. Here, there were set a group with addition of 4 µl of a 5 mg/ml aqueous solution of insulin (manufactured by TAKARA BIO INC.) as a positive control, and a group with addition of dimethyl sulfoxide as a negative control. After 45 hours, the medium was exchanged with A-D-MEM medium. In the same manner as mentioned above, the aqueous solution of the water extract fraction from *Corchorus olitorius* was added thereto so as to have a final concentration of 0.1%, or the dimethyl sulfoxide solution of the ethanol extract fraction from *Corchorus olitorius* was added thereto so as to have a final concentration of 0.067%, or the dimethyl sulfoxide solution of the ethyl acetate extract fraction from *Corchorus olitorius* was added thereto so as to have a final concentration of 0.067%, 2 µl of a 5 mg/ml aqueous solution of insulin as a positive control or dimethyl sulfoxide as a negative control was put to each well. The cells were cultured for additional 7 days. The medium was exchanged on the second day and the fourth day, and at that time an aqueous solution of the water extract fraction from *Corchorus olitorius* was added thereto so as to have a final concentration of 0.1%, or the dimethyl sulfoxide solution of the ethanol extract fraction from *Corchorus olitorius* was added thereto so as to have a final concentration of 0.067%, or the dimethyl sulfoxide solution of the ethyl acetate extract fraction from *Corchorus olitorius* was added thereto so as to have a final concentration of 0.067%, or 2 µl of a 5 mg/ml aqueous solution of insulin as a positive control, or dimethyl sulfoxide as a negative control was put to each well.

### (2) Determination of Amount of Biosynthesis of Triglyceride

The amount of triglyceride in the adipocytes was determined as an index for inducing differentiation into matured adipocytes, and as evaluation of insulin-mimetic action. After the termination of the culture, the medium was removed, and the cells were washed twice with a phosphate buffered saline. One milliliter of a solvent of hexane : isopropanol = 3 : 2 was added thereto. The mixture was allowed to stand at room temperature for 30 minutes, and thereafter the supernatant was collected. The procedures were repeated again, and 2 ml of the resulting supernatant was concentrated to dryness. The precipitate was dissolved in 100 µl of isopropanol, and thereafter the amount of triglyceride contained in 10 µl of the resulting solution was determined with Triglyceride G-Test (manufactured by Wako Pure Chemical Industries, Ltd., code 276-69801). All of the determinations were carried out twice.

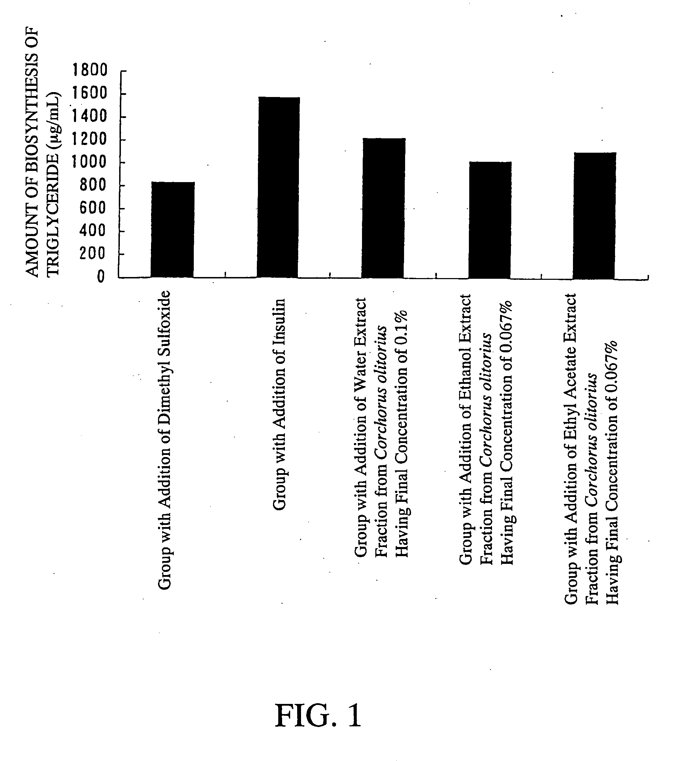

As a result, induction of triglyceride biosynthesis could be also confirmed in the group with addition of the water extract fraction from *Corchorus olitorius,* the ethanol extract fraction from *Corchorus olitorius,* or the ethyl acetate extract fraction from *Corchorus olitorius,* as compared to the group with addition of dimethyl sulfoxide, as well as in the group with addition of insulin. In other words, an activity of inducing differentiation into adipocytes was found in the water extract fraction from *Corchorus olitorius,* the ethanol extract fraction from *Corchorus olitorius,* or the ethyl acetate extract fraction from *Corchorus olitorius.* The results are shown in Figure 1. In Figure 1, the axis of abscissas is each sample, and the axis of ordinates is the amount of biosynthesis of triglyceride (µg/ml).

### Example 3 Enhancing Action by Extract Fraction from Corchorus olitorius on Glucose Uptake

### (1) Preparation of Mature Adipocytes

The induction of differentiation into mature adipocytes was carried out by partially modifying the method of Rubin C. S. et al. 3T3-L1 cells were suspended in a 10% fetal bovine serum-containing Dulbecco's modified Eagle's medium (manufactured by Sigma, D6046) containing 200 µM ascorbic acid so as to have a density of 4 × 10³ cells/ml, and the suspension was put in each well of a 12-well microtiter plate in an amount of 2 ml per well. The cells were cultured at 37°C for 7 days in the presence of 5% carbon dioxide gas. On the seventh day, the medium was exchanged with 2 mL of a 10% fetal bovine serum-containing Dulbecco's modified Eagle's medium containing 200 µM ascorbic acid, 0.25 µM dexamethasone, 10 µg/ml insulin and 0.5 mM 3-isobutyl-1-methylxanthine (manufactured by Nacalai Tesque, Inc., 19624-44). After 45 hours, the medium was exchanged with 2 mL of a 10% fetal bovine serum-containing Dulbecco's modified Eagle's medium containing 200 µM ascorbic acid and 5 µg/ml insulin. The medium was exchanged on after 2 days and 4 days, and the cells were cultured for 7 days, to give mature adipocytes.

### (2) Determination of Enhancing Action on Glucose Uptake into Mature Adipocytes

As the evaluation of enhancing action on glucose uptake, and also as the evaluation of insulin-mimetic action, the amount of 2-deoxyglucose uptake into mature adipocytes stimulated with the sample (the above-mentioned extract fraction from *Corchorus olitorius*) in the adipocytes was determined.

After the termination of the culture, the medium was removed, and the cells were washed twice with a 0.1 % (w/v) bovine serum albumin (manufactured by Sigma, A8022)-containing Dulbecco's modified Eagle's medium. Thereafter, 1 mL of the same medium containing the dimethyl sulfoxide solution of the ethanol extract fraction from *Corchorus olitorius* at a final concentration of 0.2% or 0.1%, or the dimethyl sulfoxide solution of the ethyl acetate extract fraction from *Corchorus olitorius* at a final concentration of 0.1%, each solution being prepared in Example 1, was put to each well. The cells were cultured overnight at 37°C in the presence of 5% carbon dioxide gas. Here, a group without addition of the sample was set as a negative control. After the overnight culture, the cells were washed twice with a HEPES buffered saline (140 mM NaCl, 5 mM KCl, 2.5 mM MgSO₄, 1 mM CaCl₂, 20 mM HEPES-Na (pH 7.4)), and 0.9 mL of the same buffer containing the dimethyl sulfoxide solution of the ethanol extract fraction from *Corchorus olitorius* at a final concentration of 0.2% or 0.1%, or the dimethyl sulfoxide solution of the ethyl acetate extract fraction from *Corchorus olitorius* at a final concentration of 0.1%, each solution being prepared in Example 1, was added thereto. The cells were cultured at 37°C for 75 minutes. At the point when 45 minutes passed, there was set a group with addition of insulin so as to have a final concentration of 1 µg/mL as a positive control. Thereafter, 100 µL of a HEPES buffered saline containing 2-deoxy-[1,2 ³H(N)]-glucose (manufactured by PerkinElmer Life Sciences Inc., NET549A) and 1 mM 2-deoxyglucose (manufactured by Nacalai Tesque, Inc., 10722-11) was added thereto at a final concentration of 0.5 µCi/mL, and the cells were further cultured at 37°C for 10 minutes. After the termination of the culture, the supernatant was removed, the cells were washed three times with a phosphate buffer cooled to 4°C, and 0.5 mL of a 1% Nonidet P-40-containing phosphate buffer was added to lyse the cells, whereby 2-deoxy-[1,2-³H(N)]-glucose taken into the cells was eluted. The radioactivity was determined with a liquid scintillation counter LS6500 (manufactured by Beckmann) using 25 µl of the supernatant with Aquasol-2 (manufactured by PerkinElmer Life Sciences Inc., 6NE9529) as a scintillation cocktail.

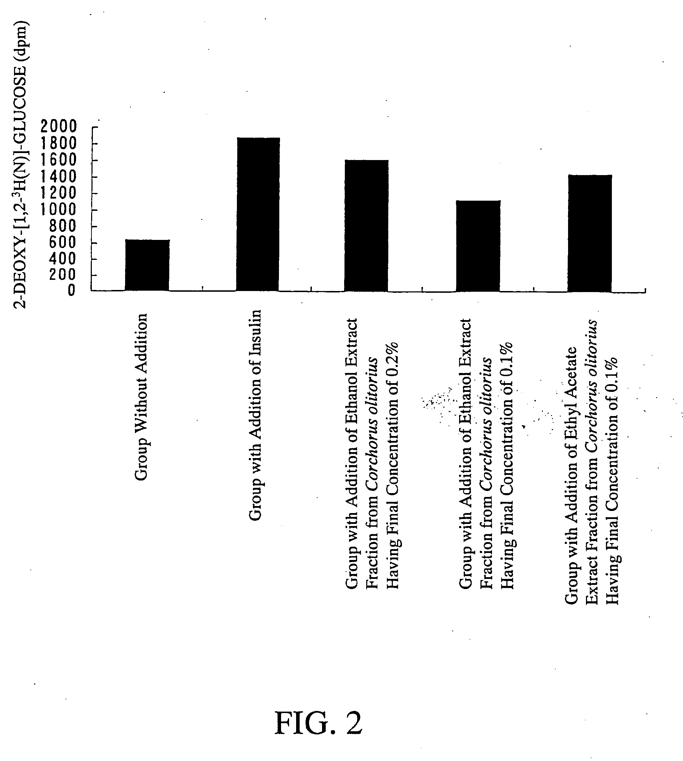

As a result, enhancement of 2-deoxy-[1,2-³H(N)]-glucose uptake was found in the group with addition of the ethanol extract fraction from *Corchorus olitorius* at each concentration or the ethyl acetate extract fraction from *Corchorus olitorius* as well as in the group with addition of insulin, as compared to the negative control. In other words, enhancing activities for glucose uptake were found in the ethanol extract fraction from *Corchorus olitorius* and the ethyl acetate extract fraction from *Corchorus olitorius.* The results are shown in Figure 2. In Figure 2, the axis of abscissas is each sample, and the axis of ordinates is the amount of 2-deoxy-[1,2-³H(N)]-glucose (dpm).

### Example 4 Preparation of Extract Fraction from Curcuma zedoaria Roscoe

Forty milliliters of distilled water, ethanol or ethyl acetate was added to 2 g each of a dry powder of *Curcuma zedoaria* Roscoe, and extraction was carried out for 30 minutes. The mixture was separated into an extract and a residue by centrifugation. Next, the extraction procedures with 30 ml of the same solvent were repeated twice for the residue. Here, the extraction with water was carried out at 60°C, and the extraction with ethanol and the extraction with ethyl acetate were carried out at room temperature. Each of these extracts obtained was combined, and the combined extract was concentrated with a rotary evaporator. Finally, a water extract was dissolved in 10 ml of distilled water, to give a water extract fraction from *Curcuma zedoaria* Roscoe. An ethanol extract was dissolved in 5 ml of dimethyl sulfoxide, to give an ethanol extract fraction from *Curcuma zedoaria* Roscoe. An ethyl acetate extract was dissolved in 5 ml of dimethyl sulfoxide, to give an ethyl acetate extract fraction from *Curcuma zedoaria* Roscoe.

### Example 5 Induction of Differentiation into Adipocytes by Extract Fraction from Curcuma zedoaria Roscoe

The inducing action on differentiation into mature adipocytes (insulin-mimetic action) of the water extract fraction from *Curcuma zedoaria* Roscoe, the ethanol extract fraction from *Curcuma zedoaria* Roscoe, and the ethyl acetate extract fraction from *Curcuma zedoaria* Roscoe, prepared in Example 4 was determined in accordance with the method of Example 2.

Specifically, as a sample, there was set a group with addition of the aqueous solution of the water extract fraction from *Curcuma zedoaria* Roscoe at a final concentration of 0.1%, the dimethyl sulfoxide solution of the ethanol extract fraction from *Curcuma zedoaria* Roscoe at a final concentration of 0.2%, 0.067%, or 0.022%, respectively, or the dimethyl sulfoxide solution of the ethyl acetate extract fraction from *Curcuma zedoaria* Roscoe at a final concentration of 0.2%, 0.067%, or 0.022%, respectively, to each well. Here, there were set a group with addition of 4 µl of a 5 mg/ml aqueous solution of insulin as a positive control, and a group with addition of dimethyl sulfoxide as a negative control. Thereafter, the medium and the sample were exchanged in the same manner as in the method described in Example 2, and after seven days from the addition of the sample, the amount of triglyceride in the adipocytes was determined.

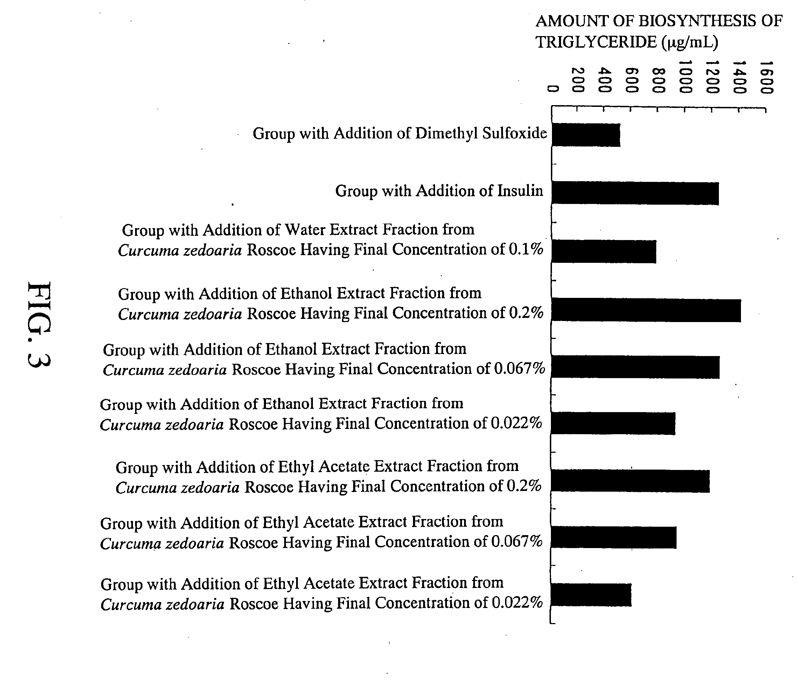

As a result, induction of biosynthesis of triglyceride was found in the groups with addition of the water extract fraction from *Curcuma zedoaria* Roscoe, and the ethanol extract fraction from *Curcumia zedoaria* Roscoe and the ethyl acetate extract fraction from *Curcuma zedoaria* Roscoe at each concentration. In other words, inducing actions on differentiation into mature adipocytes were found in the water extract fraction from *Curcuma zedoaria* Roscoe, the ethanol extract fraction from *Curcuma zedoaria* Roscoe and the ethyl acetate extract fraction from *Curcuma zedoaria* Roscoe . The results are shown in Figure 3. In Figure 3, the axis of abscissas is each sample, and the axis of ordinates is the amount of biosynthesis of triglyceride (µg/ml).

### Example 6 Enhancing Action by Extract Fraction from Curcuma zedoaria Roscoe on Glucose Uptake

The amount of 2-deoxyglucose uptake into mature adipocytes stimulated with the sample (the ethanol extract fraction from *Curcuma zedoaria* Roscoe and the ethyl acetate extract fraction from *Curcuma zedoaria* Roscoe prepared in Example 4) in the adipocytes was determined in accordance with the method described in Example 3 as the evaluation of enhancing action of the above-mentioned extract fractions from *Curcuma zedoaria* Roscoe on glucose uptake and also as the evaluation of insulin-mimetic action.

Specifically, as a sample, there was set a group with addition of the dimethyl sulfoxide solution of the ethanol extract fraction from *Curcuma zedoaria* Roscoe at a final concentration of 0.2% or 0.1%, respectively, or the dimethyl sulfoxide solution of the ethyl acetate extract fraction from *Curcuma zedoaria* Roscoe at a final concentration of 0.1% to each well. There were set a group without addition of the sample as a negative control, and a group with addition of insulin so as to have a final concentration of 1 µg/mL as a positive control. Thereafter, 2-deoxy-[1,2-³H(N)]-glucose taken into the adipocytes was determined in the same manner.

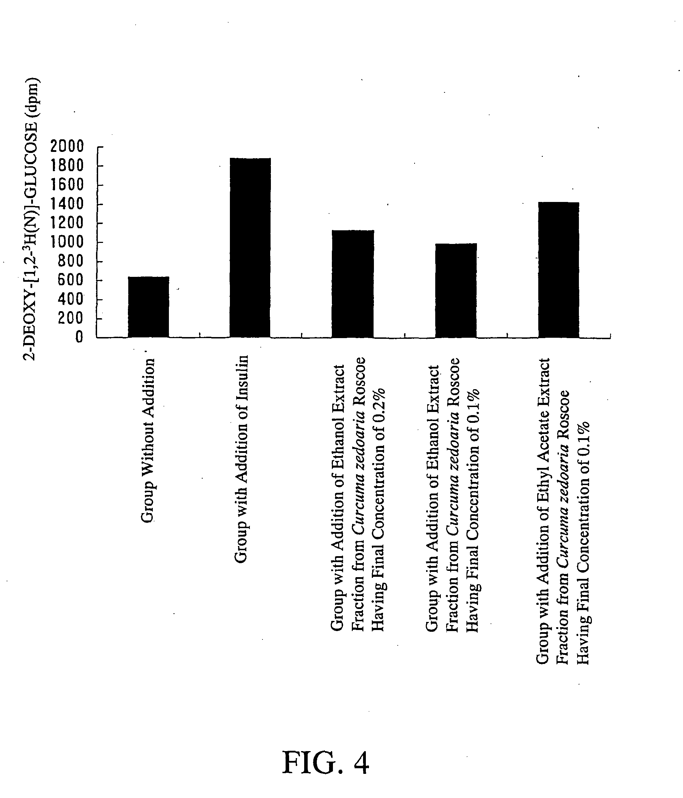

As a result, enhancement of 2-deoxy-[1,2-³H(N)]-glucose uptake was found in the groups with addition of the ethanol extract fraction from *Curcuma zedoaria* Roscoe at each concentration and the ethyl acetate extract fraction from *Curcuma zedoaria* Roscoe as well as in the group with addition of insulin, as compared to the negative control. In other words, the activities of enhancing glucose uptake were found in the ethanol extract fraction from *Curcuma zedoaria* Roscoe and the ethyl acetate extract fraction from *Curcuma zedoaria* Roscoe. The results are shown in Figure 4. In Figure 4 , the axis of abscissas is each sample, and the axis of ordinates is the amount of 2-deoxy-[1,2-³H(N)]-glucose (dpm).

### Example 7 Preparation of Extract Fraction from Chrysanthemum coronarium

Forty milliliters of distilled water, ethanol or ethyl acetate was added to 2 g each of a dry powder of *Chrysanthemum coronarium,* and extraction was carried out for 30 minutes. The mixture was separated into an extract and a residue by centrifugation. Next, the extraction procedures with 30 ml of the same solvent were repeated twice for the residue. Here, the extraction with water was carried out at 60°C, and the extraction with ethanol and the extraction with ethyl acetate were carried out at room temperature. Each of these extracts obtained was combined, and the combined extract was concentrated with a rotary evaporator. Finally, a water extract was dissolved in 10 ml of distilled water, to give a water extract fraction from *Chrysanthemum coronarium.* An ethanol extract was dissolved in 5 ml of dimethyl sulfoxide, to give an ethanol extract fraction from *Chrysanthemum coronarium.* An ethyl acetate extract was dissolved in 5 ml of dimethyl sulfoxide, to give an ethyl acetate extract fraction from *Chrysanthemum coronarium.*

### Example 8 Induction of Differentiation into Adipocytes by Extract Fraction from Chrysanthemum coronarium

The inducing action on differentiation into mature adipocytes (insulin-mimetic action) of the water extract fraction from *Chrysanthemum coronarium* prepared in Example 7 was determined in accordance with the method of Example 2.

Specifically, as a sample, there was set a group with addition of the aqueous solution of the water extract fraction from *Chrysanthemum coronarium* at a final concentration of 0.4%, 0.2%, or 0.1%, respectively, to each well. Here, there were set a group with addition of 4 µl of a 5 mg/ml aqueous solution of insulin as a positive control, and a group with addition of dimethyl sulfoxide as a negative control. Thereafter, the medium and the sample were exchanged in the same manner as in the method described in Example 2, and after seven days from the addition of the sample, the amount of triglyceride in the adipocytes was determined.

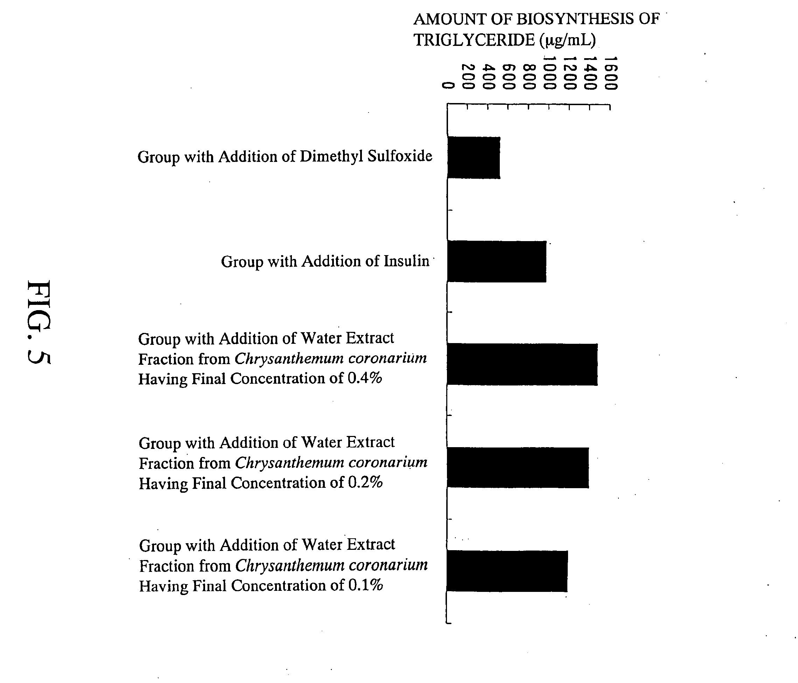

As a result, induction of biosynthesis of triglyceride was found in the group with addition of the water extract fraction from *Chrysanthemum coronarium* at each concentration. In other words, inducing action on differentiation into mature adipocytes was found in the water extract fraction from *Chrysanthemum coronarium.* The results are shown in Figure 5. In Figure 5, the axis of abscissas is each sample, and the axis of ordinates is the amount of biosynthesis of triglyceride (µg/ml).

### Example 9 Enhancing Action by Extract Fraction from Chrysanthemum coronarium on Glucose Uptake

The amount of 2-deoxyglucose uptake into mature adipocytes stimulated with the sample (the ethanol extract fraction from *Chrysanthemum coronarium* and the ethyl acetate extract fraction from *Chrysanthemum coronarium* prepared in Example 7) in the adipocytes was determined in accordance with the method described in Example 3 as the evaluation of the enhancing action of the above-mentioned extract fractions from *Chrysanthemum coronarium* on glucose uptake and also as the evaluation of the insulin-mimetic action.

Specifically, as a sample, there was set a group with addition of the dimethyl sulfoxide solution of the ethanol extract fraction from *Chrysanthemum coronarium* at a final concentration of 0.2% or 0.1%, respectively, or the dimethyl sulfoxide solution of the ethyl acetate extract fraction from *Chrysanthemum coronarium* at a final concentration of 0.1% to each well. There were set a group without addition of the sample as a negative control, and a group with addition of insulin so as to have a final concentration of 1 µg/mL as a positive control. Thereafter, 2-deoxy-[1,2-³H(N)]-glucose taken into the adipocytes was determined in the same manner.

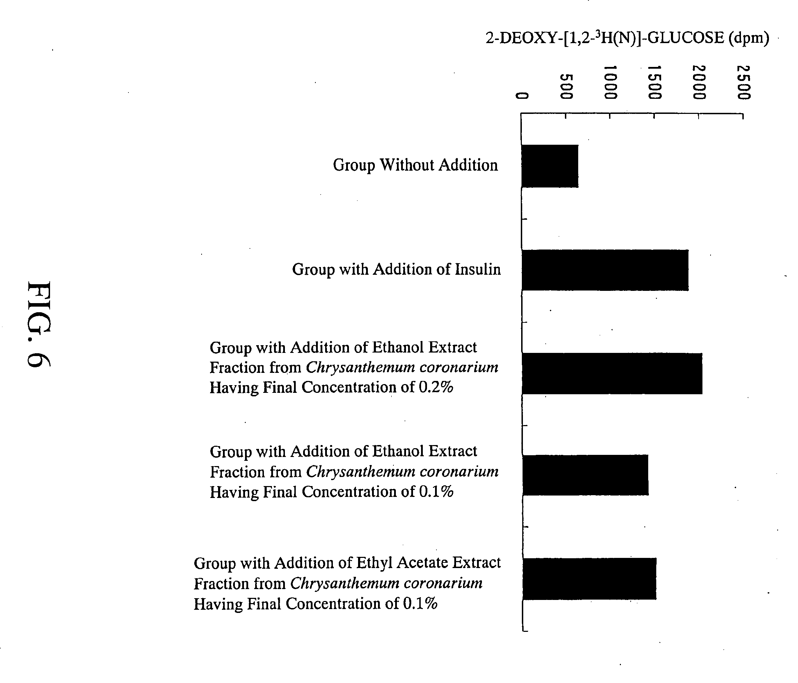

As a result, enhancement of 2-deoxy-[1,2-³H(N)]-glucose uptake was found in the groups with addition of the ethanol extract fraction from *Chrysanthemum coronarium* at each concentration and the ethyl acetate extract fraction from *Chrysanthemum coronarium* as well as in the group with addition of insulin, as compared to the negative control. In other words, activities of enhancing glucose uptake were found in the ethanol extract fraction from *Chrysanthemum coronarium* and the ethyl acetate extract fraction from *Chrysanthemum coronarium.* The results are shown in Figure 6. In Figure 6, the axis of abscissas is each sample, and the axis of ordinates is the amount of 2-deoxy-[1,2-³H(N)]-glucose (dpm).

### Example 10 Preparation of Extract Fraction from Artemisia princeps Pampan

Forty milliliters of ethanol or ethyl acetate was added to 2 g each of a dry powder of *Artemisia princeps* Pampan, and extraction was carried out at room temperature for 30 minutes. The mixture was separated into an extract and a residue by centrifugation. Next, the extraction procedures with 30 ml of the same solvent were repeated twice for the residue. Each of these extracts obtained was combined, and the combined extract was concentrated with a rotary evaporator. Finally, an ethanol extract was dissolved in 5 ml of dimethyl sulfoxide, to give an ethanol extract fraction *Artemisia princeps* Pampan. An ethyl acetate extract was dissolved in 5 ml of dimethyl sulfoxide, to give an ethyl acetate extract fraction from *Artemisia princeps* Pampan.

### Example 11 Induction of Differentiation into Adipocytes by Extract Fraction from Artemisia princeps Pampan

The inducing action on differentiation into mature adipocytes (insulin-mimetic action) of the ethanol extract fraction from *Artemisia princeps* Pampan and the ethyl acetate extract fraction from *Artemisia princeps* Pampan, prepared in Example 10 was determined in accordance with the method of Example 2.

Specifically, as a sample, there was set a group with addition of the dimethyl sulfoxide solution of the ethanol extract fraction from *Artemisia princeps* Pampan at a final concentration of 0.2%, 0.067%, or 0.022%, respectively, to each well, or addition of the dimethyl sulfoxide solution of the ethyl acetate extract fraction from *Artemisia princeps* Pampan at a final concentration of 0.2%, 0.067%, or 0.022%, respectively, to each well. Here, there were set a group with addition of 4 µl of a 5 mg/ml aqueous solution of insulin as a positive control, and a group with addition of dimethyl sulfoxide as a negative control. Thereafter, the medium and the sample were exchanged in the same manner as in the method described in Example 2, and after seven days from the addition of the sample, the amount of triglyceride in the adipocytes was determined.

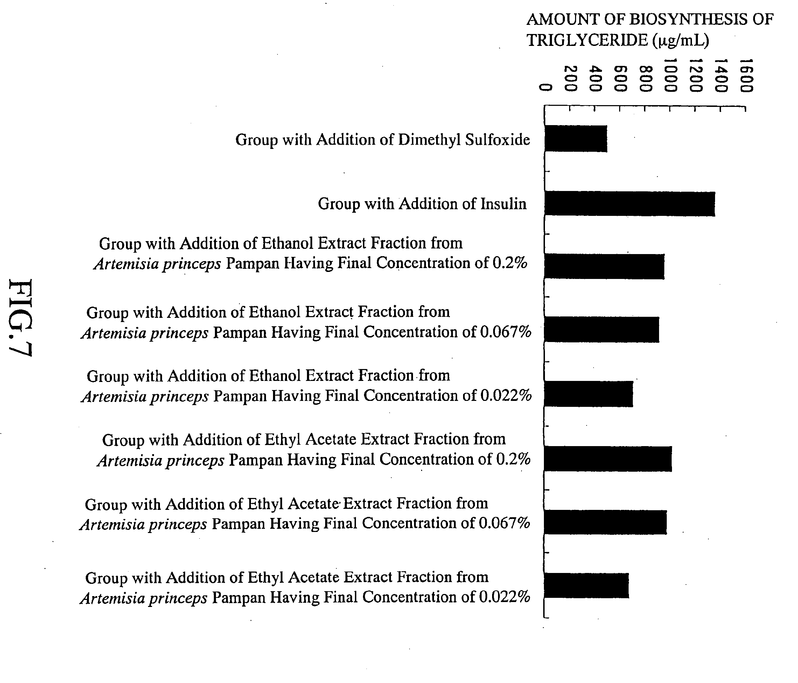

As a result, induction of biosynthesis of triglyceride was found in the groups with addition of the ethanol extract fraction from *Artemisia princeps* Pampan and the ethyl acetate extract fraction from *Artemisia princeps* Pampan at each concentration. In other words, inducing actions on differentiation into mature adipocytes were found in the ethanol extract fraction from *Artemisia princeps* Pampan and the ethyl acetate extract fraction from *Artemisia princeps* Pampan. The results are shown in Figure 7. In Figure 7, the axis of abscissas is each sample, and the axis of ordinates is the amount of biosynthesis of triglyceride (µg/ml).

### Example 12 Enhancing Action by Extract Fraction from Artemisia princeps Pampan on Glucose Uptake

The amount of 2-deoxyglucose uptake into mature adipocytes stimulated with the sample (the ethanol extract fraction from *Artemisia princeps* Pampan and the ethyl acetate extract fraction from *Artemisia princeps* Pampan prepared in Example 10) in the adipocytes was determined in accordance with the method described in Example 3 as the evaluation of enhancing action by the above-mentioned extract fractions from *Artemisia princeps* Pampan on glucose uptake and also as the evaluation of insulin-mimetic action.

Specifically, as a sample, there was set a group with addition of the dimethyl sulfoxide solution of the ethanol extract fraction from *Artemisia princeps* Pampan at a final concentration of 0.2% or 0.1%, respectively, or the dimethyl sulfoxide solution of the ethyl acetate extract fraction from *Artemisia princeps* Pampan at a final concentration of 0.1% to each well. There were set a group without addition of the sample as a negative control, and a group with addition of insulin so as to have a final concentration of 1 µg/mL as a positive control. Thereafter, 2-deoxy-[1,2-³H(N)]-glucose taken into the adipocytes was determined in the same manner.

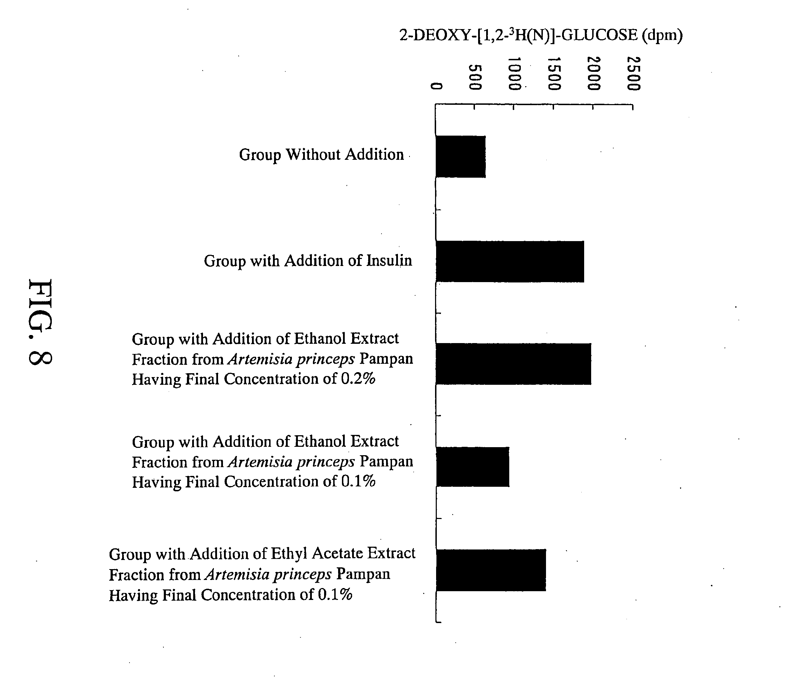

As a result, enhancement of 2-deoxy-[1,2-³H(N)]-glucose uptake was found in the groups with addition of the ethanol extract fraction from *Artemisia princeps* Pampan at each concentration and the ethyl acetate extract fraction from *Artemisia princeps* Pampan as well as in the group with addition of insulin, as compared to the negative control. In other words, the activities of enhancing glucose uptake were found in the ethanol extract fraction from *Artemisia princeps* Pampan and the ethyl acetate extract fraction from *Artemisia princeps* Pampan. The results are shown in Figure 8. In Figure 8 , the axis of abscissas is each sample, and the axis of ordinates is the amount of 2-deoxy-[1,2-³H(N)]-glucose (dpm).

### Example 13 Preparation of Extract Fraction from Aloe arborescens

Forty milliliters of ethanol or ethyl acetate was added to 2 g each of a dry powder of *Aloe arborescens,* and extraction was carried out at room temperature for 30 minutes. The mixture was separated into an extract and a residue by centrifugation. Next, the extraction procedures with 30 ml of the same solvent were repeated twice for the residue. Each of these extracts obtained was combined, and the combined extract was concentrated with a rotary evaporator. Finally, an ethanol extract was dissolved in 5 ml of dimethyl sulfoxide, to give an ethanol extract fraction *Aloe arborescens.* An ethyl acetate extract was dissolved in 5 ml of dimethyl sulfoxide, to give an ethyl acetate extract fraction from *Aloe arborescens.*

### Example 14 Enhancing Action by Extract Fraction from Aloe arborescens on Glucose Uptake

The amount of 2-deoxyglucose uptake into mature adipocytes stimulated with the sample (the ethanol extract fraction from *Aloe arborescens* and the ethyl acetate extract fraction from *Aloe arborescens* prepared in Example 13) in the adipocytes was determined in accordance with the method described in Example 3 as the evaluation of the enhancing action by the above-mentioned extract fractions from *Aloe arborescens* on glucose uptake and also as the evaluation of insulin-mimetic action.

Specifically, as a sample, there was set a group with addition of the dimethyl sulfoxide solution of the ethanol extract fraction from *Aloe arborescens* at a final concentration of 0.05%, or the dimethyl sulfoxide solution of the ethyl acetate extract fraction from *Aloe arborescens* at a final concentration of 0.05% to a well. There were set a group without addition of the sample as a negative control, and a group with addition of insulin so as to have a final concentration of 1 µg/mL as a positive control. Thereafter, 2-deoxy-[1,2-³H(N)]-glucose taken into the adipocytes was determined in the same manner.

As a result, enhancement of 2-deoxy-[1,2-³H(N)]-glucose uptake was found in the groups with addition of the ethanol extract fraction from *Aloe arborescens* and the ethyl acetate extract fraction from *Aloe arborescens* as well as in the group with addition of insulin, as compared to the negative control. In other words, the activities of enhancing glucose uptake were found in the ethanol extract fraction from *Aloe arborescens* and the ethyl acetate extract fraction from *Aloe arborescens.*

### Example 15 Preparation of Extract Fraction from Polygonum hydropiper

Forty milliliters of distilled water, ethanol or ethyl acetate was added to 2 g each of a dry powder of *Polygonum hydropiper,* and extraction was carried out for 30 minutes. The mixture was separated into an extract and a residue by centrifugation. Next, the extraction procedures with 30 ml of the same solvent were repeated twice for the residue. Here, the extraction with water was carried out at 60°C, and the extraction with ethanol and the extraction with ethyl acetate were carried out at room temperature. Each of these extracts obtained was combined, and the combined extract was concentrated with a rotary evaporator. Finally, a water extract was dissolved in 10 ml of distilled water, to give a water extract fraction from *Polygonum hydropiper.* An ethanol extract was dissolved in 5 ml of dimethyl sulfoxide, to give an ethanol extract fraction from *Polygonum hydropiper.* An ethyl acetate extract was dissolved in 5 ml of dimethyl sulfoxide, to give an ethyl acetate extract fraction from *Polygonum hydropiper.*

### Example 16 Induction of Differentiation into Adipocytes by Extract Fraction from Polygonum hydropiper

The inducing action on differentiation into mature adipocytes (insulin-mimetic action) of the water extract fraction from *Polygonum hydropiper* prepared in Example 15 was determined in accordance with the method of Example 2.

Specifically, as a sample, there was set a group with addition of the aqueous solution of the water extract fraction from *Polygonum hydropiper* at a final concentration of 0.2% or 0.1 %, respectively, to each well. Here, there were set a group with addition of 4 µl of a 5 mg/ml aqueous solution of insulin as a positive control, and a group with addition of dimethyl sulfoxide as a negative control. Thereafter, the medium and the sample were exchanged in the same manner as in the method described in Example 2, and after seven days from the addition of the sample, the amount of triglyceride in the adipocytes was determined.

As a result, induction of biosynthesis of triglyceride was found in the group with addition of the water extract fraction from *Polygonum hydropiper* at each concentration. In other words, inducing action on differentiation into mature adipocytes was found in the water extract fraction from *Polygonum hydropiper.*

### Example 17 Enhancing Action by Extract Fraction from Polygonum hydropiper on Glucose Uptake

The amount of 2-deoxyglucose uptake into mature adipocytes stimulated with the sample (the ethanol extract fraction from *Polygonum hydropiper* and the ethyl acetate extract fraction from *Polygonum hydropiper* prepared in Example 15) in the adipocytes was determined in accordance with the method described in Example 3 as the evaluation of enhancing action of the above-mentioned extract fractions from *Polygonum hydropiper* on glucose uptake and also as the evaluation of insulin-mimetic action.

Specifically, as a sample, there was set a group with addition of the dimethyl sulfoxide solution of the ethanol extract fraction from *Polygonum hydropiper* at a final concentration of 0.025%, 0.0125%, or 0.0063%, respectively, or the dimethyl sulfoxide solution of the ethyl acetate extract fraction from *Polygonum hydropiper* at a final concentration of 0.008% to each well. There were set a group without addition of the sample as a negative control, and a group with addition of insulin so as to have a final concentration of 1 µg/mL as a positive control. Furthermore, there was set a group with addition of the dimethyl sulfoxide solution of the ethanol extract fraction from *Polygonum hydropiper* at the same time as the group with addition of insulin (treatment for short period of time). In other words, there was set a group with addition of the ethanol extract fraction from *Polygonum hydropiper* so as to have a final concentration of 0.03%, 0.01%, or 0.003% in place of insulin. Thereafter, 2-deoxy-[1,2-³H(N)]-glucose taken into the adipocytes was determined in the same manner.

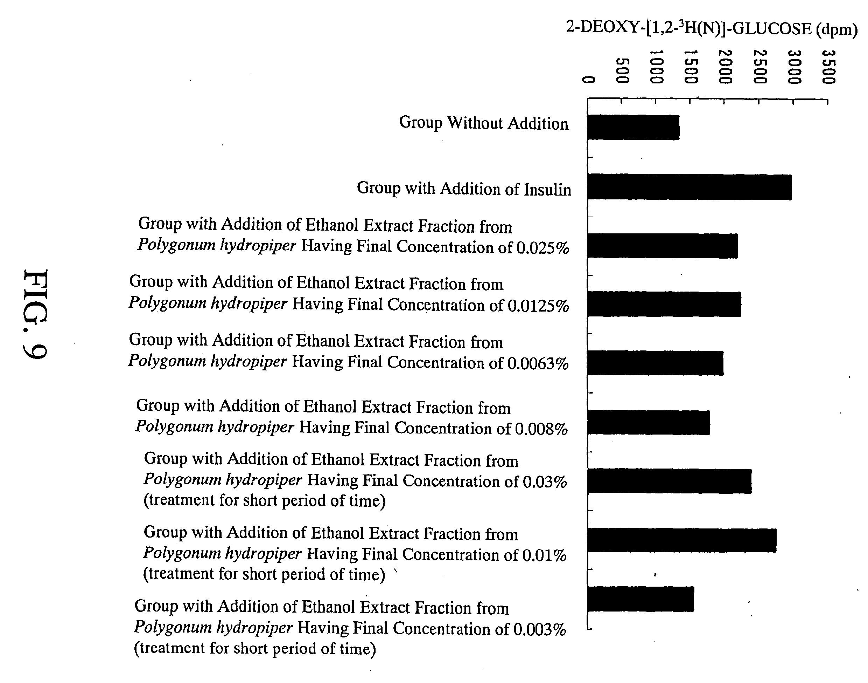

As a result, enhancement of 2-deoxy-[1,2-³H(N)]-glucose uptake was found in the group with addition of the ethanol extract fraction from *Polygonum hydropiper* at each concentration and the ethyl acetate extract fraction from *Polygonum hydropiper,* and the group with addition of the ethanol extract fraction from *Polygonum hydropiper* at each concentration simultaneously with insulin as well as in the group with addition of insulin, as compared to the negative control. In other words, activities of enhancing glucose uptake were found in the ethanol extract fraction from *Polygonum hydropiper* and the ethyl acetate extract fraction from *Polygonum hydropiper.* In addition, activities of enhancing glucose uptake were found in the ethanol extraction fraction from *Polygonum hydropiper* even in a treatment for a short period of time. The results are shown in Figure 9. In Figure 9, the axis of abscissas is each sample, and the axis of ordinates is the amount of 2-deoxy-[1,2-³H(N)]-glucose (dpm).

### Example 18 Synergistic Action of Ethanol Extract Fraction from Polygonum hydropiper and Insulin to Enhance Glucose Uptake

As the evaluation of synergistic action of the ethanol extract fraction from *Polygonum hydropiper* prepared in Example 15 and a low-concentration insulin to enhance glucose uptake, the amount of 2-deoxyglucose uptake into mature adipocytes stimulated with the sample (the above-mentioned extract fraction from *Polygonum hydropiper* and insulin) was determined partly in accordance with the method described in Example 17. The mature adipocytes were prepared in accordance with the method described in Example 3.

After the termination of the culture, the medium was removed, and the cells were washed twice with a 0.1 % (w/v) bovine serum albumin-containing Dulbecco's modified Eagle's medium. Thereafter, the cells were cultured overnight at 37°C in the presence of 5% carbon dioxide gas. After the overnight culture, the cells were washed twice with a HEPES buffered saline (140 mM NaCl, 5 mM KCl, 2.5 mM MgSO₄, 1 mM CaCl₂, 20 mM HEPES-Na (pH 7.4)). The amount 0.9 mL of the same buffer was added thereto, and the cells were cultured at 37°C for 45 minutes. Subsequently, the ethanol extract fraction from *Polygonum hydropiper* was added so as to have a final concentration of 0.013%, and further insulin was added thereto so as to have a final concentration of 0.01 µg/mL. The cells were cultured for 30 minutes. During the culture, as control, there were set a group with addition of insulin so as to have a final concentration of 0.01 µg/mL to the well without addition of the ethanol extract fraction from *Polygonum hydropiper,* and a group with addition of the ethanol extract fraction from *Polygonum hydropiper* so as to have a final concentration of 0.013% to the well without addition of insulin. In addition, a group without addition of the sample was set as a negative control. Thereafter, 2-deoxy-[1,2-³H(N)]-glucose taken into the adipocytes was determined in the same manner as the method described in Example 3.

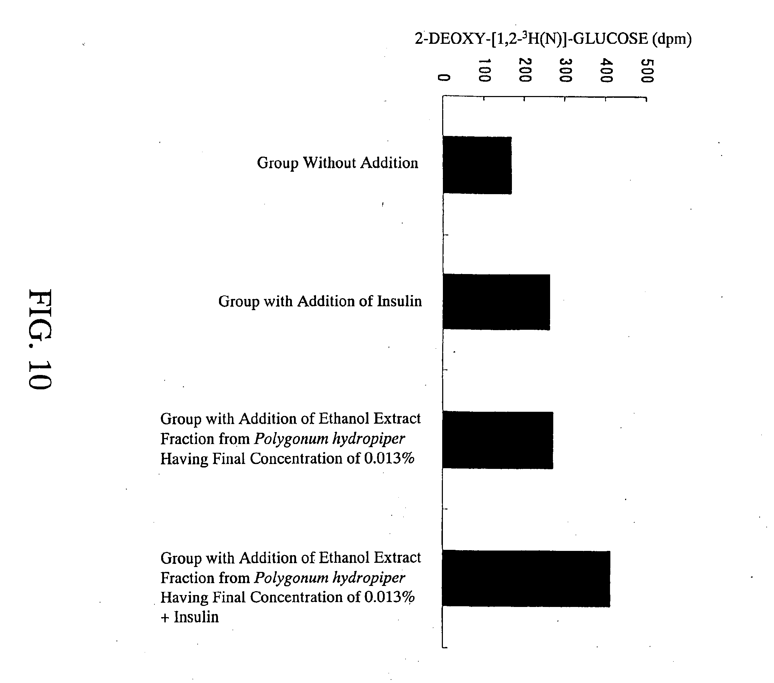

As a result, enhancement of 2-deoxy-[1,2 ³H(N)]-glucose uptake was found in the group with addition of the ethanol extract fraction from *Polygonum hydropiper* as well as in the group with addition of insulin, as compared to the negative control. Enhancement of glucose uptake was found in the group with the addition simultaneously with insulin, more than any of the group with the addition of insulin alone and the group with addition of the ethanol extract fraction from *Polygonum hydropiper* alone. In other words, an activity of enhancing glucose uptake was found to synergistically increase by the simultaneous addition of the ethanol extract fraction from *Polygonum hydropiper* with insulin. The results are shown in Figure 10. In Figure 10, the axis of abscissas is each sample, and the axis of ordinates is the amount of 2-deoxy-[1,2-³H(N)]-glucose (dpm).

### Example 19 Enhancing Action by Ethanol Extract Fraction from Polygonum hydropiper on Glucose Uptake Inhibited by Cytochalasin B

The influence of cytochalasin B on 2-deoxyglucose uptake into mature adipocytes stimulated with the sample in the adipocytes was tested in accordance with the method described in Example 17 on whether or not the enhancing action of the ethanol extract fraction from *Polygonum hydropiper* obtained in Example 15 on glucose uptake is inhibited by cytochalasin B, which is an inhibitor of a glucose transporter.

Specifically, as a sample, there was set a group with addition of a dimethyl sulfoxide solution of the ethanol extract fraction from *Polygonum hydropiper* so as to have a final concentration of 0.05% to a well (treatment for a short period of time). There were set a group without addition of the sample as a negative control, and a group with addition of insulin so as to have a final concentration of 1 µg/mL as a positive control. Furthermore, in each group, there was set a group with addition of cytochalasin B (manufactured by Nacalai Tesque, Inc., 10435-81) so as to have a final concentration of 40 µM at the same time as the time of addition of insulin or the sample. Thereafter, the amount of 2-deoxy-[1,2-³H(N)]-glucose taken into the adipocytes was determined in the same manner.

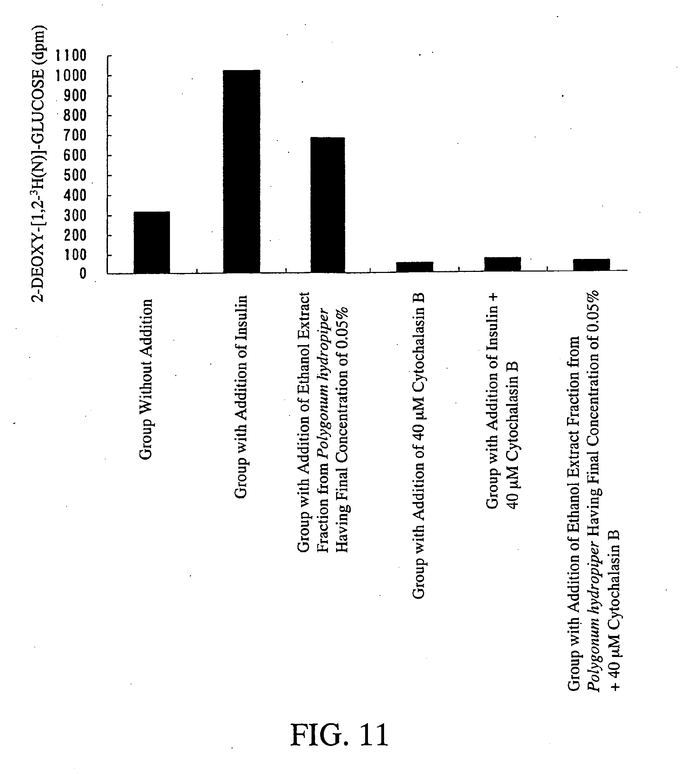

As a result, enhancement of 2-deoxy-[1,2-³H(N)]-glucose uptake was found in the group with addition of the ethanol extract fraction from *Polygonum hydropiper* as well as in the group with addition of insulin, as compared to the negative control, but 2-deoxy-[1,2-³H(N)]-glucose uptake was almost completely inhibited by addition of cytochalasin B in every group. In other words, it could be confirmed that the activity of the ethanol extract fraction from *Polygonum hydropiper* to enhance glucose uptake was mediated by a glucose transporter as well as in insulin. The results are shown in Figure 11. In Figure 11, the axis of abscissas is each sample, and the axis of ordinates is the amount of 2-deoxy-[1,2-³H(N)]-glucose (dpm).

### INDUSTRIAL APPLICABILITY

According to the present invention , there is provided a medicament, food, beverage or feed for the treatment or prevention of a disease accompanying an abnormality in an amount of insulin or insulin response, characterized in that each comprises as an effective ingredient at least one processed product derived from a plant selected from the group consisting of (a) a plant belonging to Tiliaceae, (b) a plant belonging to Zingiberaceae, (c) a plant belonging to Compositae, (d) a plant belonging to Liliaceae, and (e) a plant belonging to Polygonaceae.

The medicament is useful as a therapeutic agent or a prophylactic agent for a disease accompanying an abnormality in an amount of insulin or insulin response, such as diabetes or obesity. In addition, by taking the food or beverage as daily foodstuff, amelioration in symptoms and the like of a disease accompanying an abnormality in an amount of insulin or insulin response is enabled. Therefore, the foodstuff of the present invention comprising a processed product derived from various plants mentioned above are functional foodstuff, and are useful in maintaining homeostasis of a living body due to its an insulin-mimetic action. Also, according to the present invention, there is also provided an agent for an insulin-mimetic action comprising a processed product derived from various plants mentioned above. The agent for an insulin-mimetic action is useful in functional studies of insulin, or screening of a medicament for a disease associated with insulin. Also, according to the present invention, there is provided an agent for enhancing glucose uptake into a cell, comprising a processed product derived from various plants mentioned above. The agent for enhancing glucose uptake is also useful in treating or preventing a disease requiring the enhancing action on glucose uptake into a cell for the treatment or prevention, or preparing a food, beverage or feed for the treatment or prevention of the disease, or screening a drug for a disease requiring the enhancing action on glucose uptake. In addition, according to the present invention, there is provided an agent for inducing differentiation into an adipocyte, comprising a processed product derived from various plants mentioned above. The agent for inducing the differentiation is also useful in treating or preventing a disease requiring the inducing action on differentiation into an adipocyte for the treatment or prevention, or preparing a food, beverage or feed for the treatment or prevention of the disease, or screening a drug for a disease requiring the inducing action on the differentiation.

## Claims

1. A therapeutic agent or prophylactic agent for a disease accompanying an abnormality in an amount of insulin or insulin response, **characterized in that** the therapeutic agent or prophylactic agent comprises as an effective ingredient a processed product derived from at least one plant selected from the group consisting of (a) a plant belonging to Tiliaceae, (b) a plant belonging to Zingiberaceae, (c) a plant belonging to Compositae, (d) a plant belonging to Liliaceae, and (e) a plant belonging to Polygonaceae.

2. The therapeutic agent or prophylactic agent according to claim 1, wherein the plant belonging to Tiliaceae is *Corchorus olitorius,* the plant belonging to Zingiberaceae is *Curcuma zedoaria* Roscoe, the plant belonging to Compositae is *Chrysanthemum coronarium* and/or *Artemisia princeps* Pampan, the plant belonging to Liliaceae is *Aloe arborescens,* and the plant belonging to Polygonaceae is *Polygonum hydropiper.*

3. An agent for an insulin-mimetic action, **characterized in that** the agent for an insulin-mimetic action comprises as an effective ingredient a processed product derived from at least one plant selected from the group consisting of (a) a plant belonging to Tiliaceae, (b) a plant belonging to Zingiberaceae, (c) a plant belonging to Compositae, (d) a plant belonging to Liliaceae, and (e) a plant belonging to Polygonaceae.

4. The agent for an insulin-mimetic action according to claim 3, wherein the plant belonging to Tiliaceae is *Corchorus olitorius,* the plant belonging to Zingiberaceae is *Curcuma zedoaria* Roscoe, the plant belonging to Compositae is *Chrysanthemum coronarium* and/or *Artemisia princeps* Pampan, the plant belonging to Liliaceae is *Aloe arborescens,* and the plant belonging to Polygonaceae is *Polygonum hydropiper.*

5. A food, beverage or feed for treating or preventing a disease accompanying an abnormality in an amount of insulin or insulin response, **characterized in that** the food, beverage or feed comprises as an effective ingredient a processed product derived from at least one plant selected from the group consisting of (a) a plant belonging to Tiliaceae, (b) a plant belonging to Zingiberaceae, (c) a plant belonging to Compositae, (d) a plant belonging to Liliaceae, and (e) a plant belonging to Polygonaceae.

6. The food, beverage or feed according to claim 5, wherein the plant belonging to Tiliaceae is *Corchorus olitorius,* the plant belonging to Zingiberaceae is *Curcuma zedoaria* Roscoe, the plant belonging to Compositae is *Chrysanthemum coronarium* and/or *Artemisia princeps* Pampan, the plant belonging to Liliaceae is *Aloe arborescens,* and the plant belonging to Polygonaceae is *Polygonum hydropiper.*

7. An agent for enhancing glucose uptake into a cell, **characterized in that** the agent for enhancing glucose uptake comprises as an effective ingredient a processed product derived from at least one selected from the group consisting of (a) a plant belonging to Tiliaceae, (b) a plant belonging to Zingiberaceae, (c) a plant belonging to Compositae, (d) a plant belonging to Liliaceae, and (e) a plant belonging to Polygonaceae.

8. The agent for enhancing glucose uptake according to claim 7, wherein the plant belonging to Tiliaceae is *Corchorus olitorius,* the plant belonging to Zingiberaceae is *Curcuma zedoaria* Roscoe, the plant belonging to Compositae is *Chrysanthemum coronarium* and/or *Artemisia princeps* Pampan, the plant belonging to Liliaceae is *Aloe arborescens,* and the plant belonging to Polygonaceae is *Polygonum hydropiper.*

9. An agent for inducing differentiation into an adipocyte, **characterized in that** the agent for inducing differentiation comprises as an effective ingredient a processed product derived from at least one plant selected from the group consisting of (a) a plant belonging to Tiliaceae, (b) a plant belonging to Zingiberaceae, (c) a plant belonging to Compositae, (d) a plant belonging to Liliaceae, and (e) a plant belonging to Polygonaceae.

10. The agent for inducing differentiation into an adipocyte according to claim 9, wherein the plant belonging to Tiliaceae is *Corchorus olitorius,* the plant belonging to Zingiberaceae is *Curcuma zedoaria* Roscoe, the plant belonging to Compositae is *Chrysanthemum coronarium* and/or *Artemisia princeps* Pampan, the plant belonging to Liliaceae is *Aloe arborescens,* and the plant belonging to Polygonaceae is *Polygonum hydropiper.*
